(19) Europäisches Patentamt
European Patent Office
Office européen des brevets

(11) **EP 3 117 769 A1**

(12) **EUROPEAN PATENT APPLICATION**
published in accordance with Art. 153(4) EPC

(43) Date of publication:
**18.01.2017  Bulletin 2017/03**

(51) Int Cl.:
***A61B 5/1455*** *(2006.01)*

(21) Application number: **15761528.7**

(86) International application number:
**PCT/JP2015/055765**

(22) Date of filing: **27.02.2015**

(87) International publication number:
**WO 2015/137151 (17.09.2015 Gazette 2015/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority:  **11.03.2014  JP 2014047755**

(71) Applicant: **Nihon Kohden Corporation**
**Shinjyuku-ku**
**Tokyo 161-8560 (JP)**

(72) Inventors:
• **MORIMURA, Naoto**
  **Yokohama-shi**
  **Kanagawa 232-0024 (JP)**
• **KOBAYASHI, Naoki**
  **Tokyo 161-8560 (JP)**

• **HORIE, Katsuyuki**
  **Tokyo 161-8560 (JP)**
• **HIRABARA, Hideaki**
  **Tokyo 161-8560 (JP)**
• **SUZUKI, Katsuyoshi**
  **Tokyo 161-8560 (JP)**
• **MATSUZAWA, Wataru**
  **Tokyo 161-8560 (JP)**
• **KUMAGAI, Sou**
  **Tokyo 161-8560 (JP)**
• **TONE, Katsuhide**
  **Tokyo 161-8560 (JP)**
• **NOMURA, Kenichi**
  **Tokyo 161-8560 (JP)**

(74) Representative: **Hoffmann Eitle**
**Patent- und Rechtsanwälte PartmbB**
**Arabellastraße 30**
**81925 München (DE)**

(54) **SUPPORT TOOL AND BIOLOGICAL INFORMATION ACQUISITION SYSTEM**

(57)     A support tool (3) is mounted on a finger (5) of a subject on whom a sensor (2) is mounted to support acquisition of biological information. A first support (31) is disposed on a nail side (5a) of the subject's finger. A second support (32) is disposed on a ventral side (5b) of the subject's finger. The relative position of the second support (32) with respect to the first support (31) does not change. A bag (33) is formed with a material softer than the first support (31) and second support (32), and is disposed along an inner peripheral face of the first support (31). A ventilation tube (34) forms a ventilation channel communicating with an inside of the bag (33). The sensor (2) includes a light emission part (21) and a light reception part (22). The light emission part (21) is disposed between the subject's finger (5) and the bag (33). The light reception part (22) is disposed between the subject's finger (5) and the second support (32).

*FIG. 2*

## Description

### Technical Field

The present invention relates to a tool that supports an acquisition of biological information of a living body to which a sensor is mounted. The present invention also relates to a system that uses the tool to acquire biological information of a subject.

### Background Art

Measurement of a capillary refill time is an example of acquisition of such biological information. The capillary refill time is used as a simple index for evaluating presence/absence of a shock. The capillary refill time is a measure generally used in an emergency medical care field for judgment of necessity of transfusion, triage (priority evaluation in a mass casualty situation), etc. Specifically, a medical personnel compresses a living tissue such as a fingernail of a subject, and visually checks a change in the nail or skin color after releasing the compression. If the color returns to its original color within nearly two seconds, it is judged that the living tissue is in a normal state. Since this technique is performed by compressing a living tissue by a hand and visually checking a change in skin color, it lacks quantitativity and a measurer-dependent error may easily occur.

To solve the problem, use of a pulse oximeter for measurement of the capillary refill time has been proposed. During measurement, a support tool having an optical sensor and an actuator (a solenoid etc.) is mounted on a fingertip of a subject (see, e.g., Patent Document 1). Light having a wavelength which is absorbed by blood is made incident on a living tissue such as a fingertip and an intensity of the light transmitted through the living tissue is measured by the optical sensor. When the living tissue is compressed by the actuator, blood is removed from the living tissue at the compressed location. Hence, the intensity of the transmitted light increases. When the compression is released, the living tissue at that location is refilled with blood. Hence, the intensity of the transmitted light decreases. A capillary refill time is specified based on a time from the release of compression until the intensity of the transmitted light returns to its original level.

### Prior Art Document

### Patent Document

Patent Document 1: US Patent No. 8,082,017 B2

### Summary of Invention

### Problem to be Solved by Invention

To perform reproducible and quantitative measurement, it is necessary to fix the compression state of the living tissue. However, with the configuration in which the living tissues is compressed directly and locally by the actuator as described in Patent Document 1, it is difficult to mount the support tool on the subject's finger such that the actuator abut against the living tissue always at the same location. When the abutment position varies, the compression state changes. Therefore, reproducibility of a measurement result is lowered.

Accordingly, it is an object of the present invention to make a specific compression state easily reproducible when acquiring biological information with a compression of a living tissue.

### Solution to Problem

To achieve the object described above, a first aspect of the present invention provides a support tool to be mounted on a finger of a subject on which a sensor is mounted so as to support an acquisition of biological information of the subject, The support tool includes a first support to be disposed, when mounted, on a first side of the finger of the subject, the first support having an inner peripheral face, a second support to be disposed, when mounted, on a second side of the finger of the subject opposite to the first side, and a relative position of the second support with respect to the first support being fixed, a bag made of a material softer than the first support and the second support, the bag being at least partially disposed along the inner peripheral face and a channel formation part forming a fluid channel communicating with an inside of the bag. The support tool is configured such that, when mounted, the sensor is disposed at at least one of between the finger of the subject and the bag and between the finger of the subject and the second support.

According to this configuration, the bag made of the softer material can be inflated to compress the subject's finger, so that uniform compression force can be applied non-locally. Accordingly, even if the relative position of the support tool with respect to the subject's finger changes whenever the support tool is mounted, the influence of the change of the relative position on the compression state can be suppressed.

In addition, the sensor is mounted on the subject's finger outside the bag. Therefore, the relative position of the sensor with respect to the subject's finger does not change in accordance with inflation and deflation of the bag. Accordingly, even if the relative position of the support tool with respect to the subject's finger changes whenever the support tool is mounted, the influence of the change of the relative position on a detection result obtained by the sensor can be suppressed.

In addition, a pressurized state can be set or released by only sending air into the bag or releasing air from the bag. Therefore, it is not necessary to build an actuator driving mechanism in the support tool. Accordingly, the size and weight of the support tool can be reduced, and a burden imposed on the subject on whom the support tool is mounted can be suppressed. Further, it is possible to provide such a support tool at low cost.

The biological information may include at least one of a capillary refill time and a blood substance concentration.

As described above, according to the aforementioned configuration, a specific compression state can be reproduced easily when acquiring the biological information with a compression of a living tissue. Thus, the biological information of the subject can be acquired quantitatively and reproducibly.

The inner peripheral face may be configured to extend in an arc shape.

According to this configuration, a pressurized state can be formed by a smaller volume change of the bag, in comparison with a configuration in which a bag is mounted on a flat inner peripheral face. In other words, the pressurized state can be formed by use of the bag having a smaller volume. That is, the pressurized state can be formed rapidly even by use of a smaller-sized air-sending mechanism. A similar effect can be obtained also when the bag is deflated to release the pressurized state. Accordingly, the size of the apparatus can be reduced or the cost can be suppressed while a specific compression state of the subject's finger can be reproduced easily.

A pressing member that resembles a finger of a living body may be provided on an outer face of the bag.

According to this configuration, a more natural compression state similar to compression which has been heretofore performed by a hand of a medical personnel can be formed. Accordingly, the biological information can be acquired more naturally while a specific compression state of the subject's finger can be reproduced easily.

A fixation assistance part may be provided on at least one of the first support and the second support to assist a fixation to the finger of the subject.

According to this configuration, a user can mount the support tool on the subject's finger with the fixation assistance part as a guide. When the fixation assistance part is fixed to the finger by a tape etc. if necessary, dislocation of the support tool with respect to the finger during measurement can be prevented. Accordingly, a specific compression state of the finger can be reproduced more easily. Thus, the biological information of the subject can be acquired more reproducibly.

The fixation assistance part may have an elastic member that surrounds a part of the finger of the subject.

According to this configuration, the support tool can be fixed to the subject's finger without requiring an auxiliary fixation piece such as a tape. Thus, dislocation of the support tool with respect to the finger during measurement can be prevented so that a specific compression state of the finger can be reproduced more easily. Accordingly, the biological information of the subject can be acquired more reproducibly.

The channel formation part may be provided on an outer peripheral face of the first support and may have a connection portion to be connected to a tube communicating with the fluid channel.

According to this configuration, the support tool can be attached to the tube removably. Thus, it is not necessary to mold the tube integrally with the bag so that the manufacturing cost of the support tool can be suppressed. Accordingly, it is possible to meet a request to make only the support tool disposable while reproducing a specific compression state of the subject's finger easily.

At least one of the channel formation part and the connection portion may be movable relative to the first support.

According to this configuration, even when the tube connected to the connection portion is displaced due to body motion of the subject, the channel formation part is displaced following the body motion. Therefore, dislocation of the support tool caused by the body motion can be suppressed. Accordingly, a specific compression state of the subject's finger can be reproduced more easily. Thus, the biological information of the subject can be acquired more reproducibly.

A through hole allowing an access to the finger of the subject may formed through the first support or the second support.

According to this configuration, even in an emergency in which the mechanism for sending air into the bag cannot function for some reason, the subject's finger can be compressed directly or indirectly by a user's hand so that the biological information can be acquired.

To achieve the aforementioned object, a second aspect of the present invention provides a support tool to be mounted on a finger of a subject to support an acquisition of biological information of the subject. The support tool includes a first support to be disposed, when mounted, on a first side of the finger of the subject, the first support having an inner peripheral face, a second support to be disposed, when mounted, on a second side of the finger of the subject opposite to the first side, a relative position of the second support with respect to the first support being fixed, a bag made of a material softer than the first support and the second support, the bag being at least partially disposed along the inner peripheral face, a channel formation part forming a fluid channel communicating with an inside of the bag, and a sensor that outputs a signal corresponding to the biological information of the subject, the sensor being disposed, when mounted, at at least one of between the finger of the subject and the bag and between the finger of the subject and the second support.

According to this configuration, the biological information can be acquired by only an operation of mounting the support tool on the subject's finger, in addition to the effects which have been described about the first mode. Accordingly, the biological information of the subject can be acquired simply and reproducibly while a specific compression state of the subject's finger can be reproduced easily.

The biological information may include at least one of a capillary refill time and a blood substance concentration.

The support tool may include a tube connected to the channel formation part and having higher softness than the first support and the second support. The tube includes a ventilation channel communicating with the channel formation part to form a part of the fluid channel, and a signal line housing part that houses a signal line electrically connected to the sensor.

Alternatively, the support tool may include a tube connected to the channel formation part and having higher softness than the first support and the second support. The tube includes a ventilation channel which communicating with the channel formation part to form a part of the fluid channel, and a signal line retention part that retains, in a removable manner, a signal line electrically connected to the sensor.

According to these configurations, an electric system cable and a ventilation system hose can be combined into a single line. Thus, dislocation of the support tool due to body motion etc. of the subject can be suppressed, in comparison with a case where two lines may be displaced individually due to the body motion etc. Accordingly, a specific compression state of the subject's finger can be reproduced more easily. Thus, the biological information of the subject can be acquired more reproducibly.

To achieve the aforementioned object, a third aspect of the present invention provides a biological information acquisition system including a support tool to be mounted on a finger of a subject, a sensor to be mounted on the finger of the subject to output a signal corresponding to biological information of the subject, an information acquisition part which acquires the biological information based on the signal output from the sensor, and an information output part which outputs the information acquired by the information acquisition part. The support tool includes a first support to be disposed, when mounted, on a first side of the finger of the subject, the first support having an inner peripheral face, a second support to be disposed, when mounted, on a second side of the finger of the subject opposite to the first side, a relative position of the second support with respect to the first support being fixed, a bag made of a material softer than the first support and the second support, the bag being at least partially disposed along the inner peripheral face, and a channel formation part forming a fluid channel communicating with an inside of the bag. The sensor is disposed, when the support tool is mounted, at at least one of between the finger of the subject and the bag and between the finger of the subject and the second support. The biological information acquisition system further includes a pressure adjustment part which adjusts, through the channel formation part, a volume of a fluid inside the bag to adjust a pressure applied to the finger of the subject.

According to this configuration, the effects which have been described about the first mode and the second mode can be obtained.

The pressure adjustment part may include a motor pump.

According to this configuration, the bag can be inflated to compress the subject's finger automatically, so that a specific compression state can be reproduced easily. Thus, the biological information of the subject can be acquired quantitatively and reproducibly.

The support tool may include a switch which is actuated when mounted on the finger of the subject. In this case, the biological information acquisition system includes a control part which drives the motor pump in accordance with the actuation of the switch.

According to this configuration, a pressurization operation can be started by the motor pump automatically when the support tool is mounted on the subject's finger. In addition, in the case where the support tool is not mounted on the subject's finger in a proper position, the pressurization operation can be prevented from being started by the motor pump. Or in the case where the support tool mounted on the subject's finger is dislocated from the proper position during measurement, the pressurization operation performed by the motor pump can be suspended. Accordingly, a specific compression state of the subject's finger can be reproduced more easily. Thus, the biological information of the subject can be acquired more accurately and reproducibly.

The biological information acquisition system may include a power supply monitoring part which monitors a power feeding performance from a power supply to the motor pump. In this case, the power supply monitoring part controls the pressure adjustment part to stop driving the motor pump when the power feeding performance is lower than a predetermined value.

When the motor pump is driven under a circumstance that electric power is fed insufficiently, a desired compression state of the subject's finger may not be able to be obtained. According to the aforementioned configuration, a situation that measurement is made in this state to acquire inaccurate biological information of the subject can be avoided. Accordingly, the biological information of the subject can be acquired more accurately and reproducibly while a specific compression state of the subject's finger can be reproduced easily.

The pressure adjustment part may include a mechanism to manually adjust the volume of the fluid.

In the case of a configuration in which the manual adjustment mechanism is provided in place of the motor pump, the configuration of the pressure adjustment part can be simplified and the size and weight of the apparatus can be reduced. In the case of a configuration in which the manual adjustment mechanism is provided in addition to the motor pump, the biological information can be acquired manually even when the power feeding performance of the power supply to the motor pump becomes insufficient. When the power supply is a battery, the biological information can be acquired manually if necessary. As a result, electric power can be saved. Accordingly, the biological information of the subject can be measured in accordance with the circumstance while a specific compression state of the subject's finger can be reproduced easily.

The biological information acquisition system may include a pressure sensor which detects a pressure applied to the finger of the subject, and an audio output part which outputs a sound corresponding to the pressure.

According to this configuration, a user can easily grasp whether the subject's finger is in a compression state or not. Particularly in the case where pressurization of the bag is performed manually, the user can use sound output by the audio output part, as a guide for forming a desired compression state. Accordingly, the biological information of the subject can be acquired accurately and reproducibly while a specific compression state of the subject's finger can be reproduced more easily.

The sensor may include a light emitter which emits first light having a first wavelength, and second light having a second wavelength, and a light receiver which outputs a first signal and a second signal respectively in accordance with an intensity of the first light and an intensity of the second light which have been transmitted through or reflected on the finger of the subject. The information acquisition part may include
a first light attenuation acquisition portion which acquires a light attenuation of the first light based on the first signal, and acquires a light attenuation of the second light based on the second signal, a second light attenuation acquisition portion which acquires a blood-based light attenuation based on the light attenuation of the first light and the light attenuation of the second light, and a capillary refill time specifying portion which specifies a capillary refill time in a tissue of the finger based on a temporal change of the blood-based light attenuation due to a compression of the finger of the subject by the bag.

According to this configuration, the bag can be inflated to reproduce a specific compression state of the subject's finger easily. Accordingly, the capillary refill time of the subject can be measured quantitatively and reproducibly.

The light emitter may emit third light having a third wavelength. The light receiver may output a third signal in accordance with an intensity of the third light which has been transmitted through or reflected on the finger of the subject. The first wavelength and the second wavelength are wavelengths which are absorbed by hemoglobin. The third wavelength is a wavelength at which an absorption by water is larger than an absorption by hemoglobin. The information acquisition part may acquire information on a non-blood tissue of the subject based on at least a temporal change of the third signal generated due to the compression of the finger of the subject by the bag.

According to this configuration, the bag can be inflated to reproduce a specific compression state of the subject's finger easily. Accordingly, edema can be diagnosed reproducibly.

The sensor may include a light emitter which emits lights having N kinds of wavelengths that are different from one another, N being an integer equal to or greater than 3, and a light receiver which outputs N kinds of signals in accordance with intensities of the N kinds of light which have been transmitted through or reflected on the finger of the subject. The information acquisition part may include a first light attenuation acquisition portion which acquires N kinds of light attenuations based on the N kinds of signals, a second light attenuation acquisition portion which acquires at most N-1 kinds of blood-based light attenuations based on at most N-1 kinds of combinations of two light attenuations selected from the N kinds of light attenuations, and a blood substance concentration specifying portion which specifies at most N-1 kinds of blood substance concentrations based on the at most N-1 kinds of blood-based light attenuations.

According to this configuration, the bag can be inflated to reproduce a specific compression state of the subject's finger easily. Accordingly, the blood substance concentrations of the subject can be measured quantitatively and reproducibly.

The blood substance concentrations may be output by the information output part in a state in which the finger of the subject is not compressed by the bag. In this case, while the finger of the subject is being compressed by the bag, the information output part either suspends outputting the blood substance concentrations or keeps pre-pressurization output values of the blood substance concentrations.

According to this configuration, effective measurement values can be always presented to the user with a feeling of security while the blood substance concentrations of the subject can be measured quantitatively and reproducibly.

The biological information acquisition system may include a control part which operates the pressure adjustment part at a predetermined interval.

According to this configuration, the bag can be inflated to compress the subject's finger automatically whenever a predetermined time elapses. On this occasion, a specific compression state can be reproduced easily. Therefore, for example, patient's therapy effect or deteriorating tendency of patient's condition during an operation or in an intensive care unit (ICU) can be checked automatically and accurately.

The biological information acquisition system may include a switch which starts an operation of the pressure adjustment part, and a prohibition control part which invalidates an operation of the switch until a predetermined time elapses after the compression of the finger of the subject by the bag is released.

A certain amount of time is required until blood returns to living tissues of the finger to restore the subject's finger to its original state after the compression of the subject's finger performed by the bag is released. When the switch is operated prior to the restoration, the biological information of the subject obtained through the compression performed in accordance with the operation may lack accuracy. According to the aforementioned configuration, next measurement can be prevented from being carried out before the subject's finger which has been released from compression is restored to its original state. Accordingly, the biological information of the subject can be acquired more accurately and reproducibly while a specific compression state of the subject's finger can be reproduced easily.

The predetermined time may be variable based on an operating state of the pressure adjustment part.

When the compression is performed by the bag repeatedly, a burden imposed on the living tissues of the subject's finger becomes large in spite of non-local pressure. According to the aforementioned configuration, the burden imposed on the subject can be suppressed while a specific compression state of the subject's finger can be reproduced easily.

The biological information acquisition system may include a pressure sensor which detects an internal pressure of the bag. In this case, the pressure adjustment part adjusts the volume of the fluid inside the bag based on a detection result of the pressure sensor so that the internal pressure becomes a target value.

Even when the pressure adjustment part performs a fixed operation, pressure applied to the subject's finger may vary according to the shape of the subject's finger or the mounting positions of the sensor and the support tool. According to the aforementioned configuration, it is possible to grasp to what extent pressure has been actually applied to the subject's finger. Therefore, a specific compression state can be reproduced easily regardless of the shape of the subject's finger or the mounting positions of the sensor and the support tool. Thus, the biological information of the subject can be acquired more accurately and reproducibly.

**Brief Description of Drawings**

Fig. 1 is a functional block diagram showing the configuration of a biological information acquisition system according to an embodiment of the invention;

Fig. 2 is a sectional view showing a state in which a bag has inflated in a support tool belonging to the biological information acquisition system;

Fig. 3 is a view for illustrating an effect which can be obtained by the configuration of the support tool;

Fig. 4 is a graph illustrating an example of processing performed by a second light attenuation acquisition portion in the biological information acquisition system;

Fig. 5 is a sectional view schematically showing a modification of the support tool;

Fig. 6 is a sectional view schematically showing a modification of the support tool;

Fig. 7 is a sectional view schematically showing an example of a tube connected to the support tool;

Fig. 8 is a sectional view schematically showing another example of the tube connected to the support tool;

Fig. 9 is a perspective view showing a first embodiment of the support tool;

Fig. 10 is a perspective view showing a second embodiment of the support tool;

Fig. 11 is a perspective view showing a third embodiment of the support tool;

Fig. 12 is a perspective view showing a fourth embodiment of the support tool;

Fig. 13 is a perspective view showing a fifth embodiment of the support tool;

Fig. 14 is a sectional view taken along a line XIV-XIV of Fig. 13;

Fig. 15 is a perspective view showing a sixth embodiment of the support tool; and

Fig. 16 is a perspective view showing a seventh embodiment of the support tool.

**Embodiments of Invention**

Embodiments of the present invention will be described below in detail by way of example with reference to the accompanying drawings. In the drawings used in the following description, scales are adjusted as appropriate for the purpose of illustrating each member in a recognizable size.

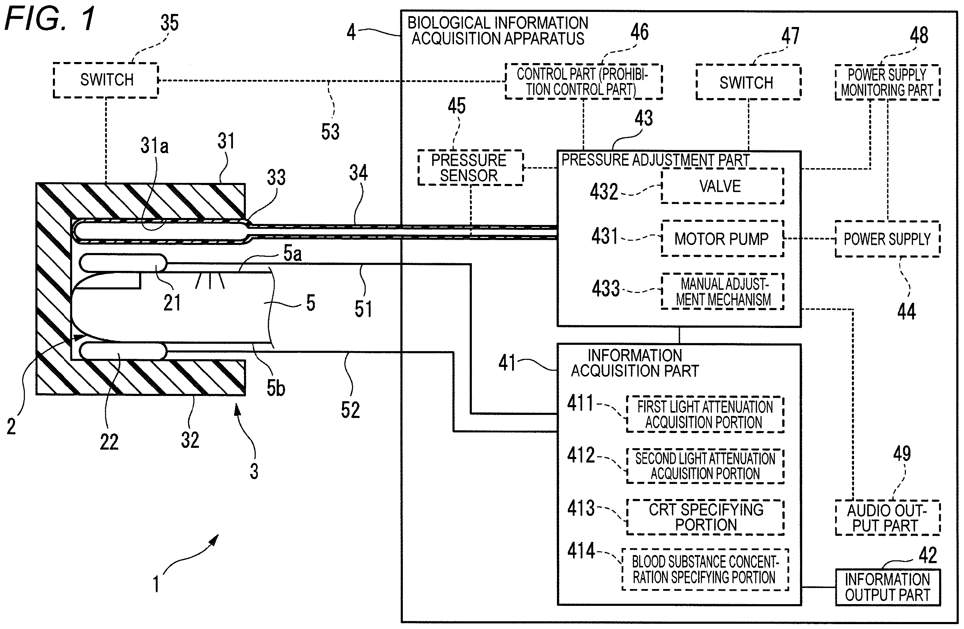

Fig. 1 is a diagram schematically showing a configuration of a biological information acquisition system 1 according to an embodiment of the present invention. The biological information acquisition system 1 includes a sensor 2, a support tool 3, and a biological information acquisition apparatus 4.

The sensor 2 is configured to be mounted on a finger 5 of a subject and output a signal corresponding to biological information of the subject.

The support tool 3 is a tool which is mounted on the finger 5 of the subject on which the sensor 2 is mounted so as to support acquisition of the biological information.

The biological information acquisition apparatus 4 includes an information acquisition part 41. The information acquisition part 41 is configured to acquire the biological information of the subject based on the signal output from the sensor 2.

The biological information acquisition apparatus 4 includes an information output part 42. The information output part 42 is configured to output the information acquired by the information acquisition part 41.

The support tool 3 includes a first support 31. The first support 31 is disposed on a nail side 5a (an example of a first side) of the finger 5 when the support tool 3 is mounted on the subject's finger 5.

The support tool 3 includes a second support 32. The second support 32 is disposed on a ventral side 5b (an example of a second side) of the finger 5 when the support tool 3 is mounted on the subject's finger 5. That is, the second support 32 is disposed on the opposite side to the first support 31 with the interposition of the finger 5 therebetween.

For example, the first support 31 and the second support 32 are molded out of a hard resin. Thus, the relative position of the first support 31 with respect to the second support 32 is set not to change.

The support tool 3 includes a bag 33. The bag 33 is made of a material softer than the first support 31 and the second support 32. For example, the material may be a soft resin, rubber, or the like. The bag 33 is disposed along an inner peripheral face 31a of the first support 31.

In a state in which the support tool 3 is mounted on the subject's finger 5, the sensor 2 is disposed between the subject's finger 5 and the second support 32 and between the subject's finger 5 and the bag 33.

The support tool 3 includes a ventilation tube 34 (an example of a channel formation part). The ventilation tube 34 forms a ventilation channel (an example of a fluid channel) communicating with the inside of the bag 33.

The biological information acquisition apparatus 4 includes a pressure adjustment part 43. The pressure adjustment part 43 is configured to adjust the volume of air inside the bag 33 through the ventilation tube 34 to thereby adjust pressure applied to the subject's finger 5.

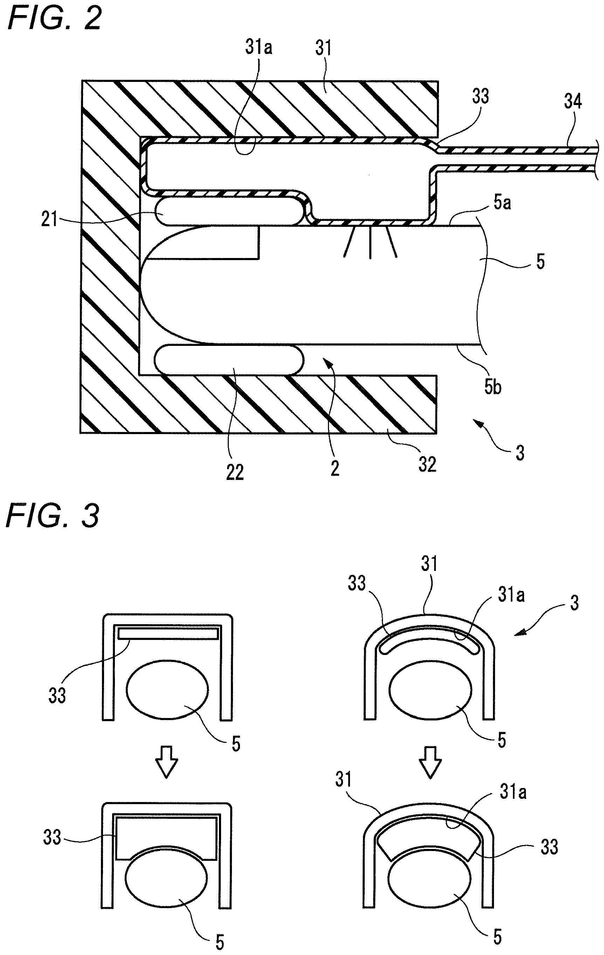

Specifically, the pressure adjustment part 43 sends air into the bag 33 through the ventilation tube 34 to thereby increase the volume of air inside the bag 33. Thus, as shown in Fig. 2, the bag 33 made of a soft material inflates to compress the subject's finger 5 together with the sensor 2. Due to this compression, blood in living tissues of the subject's finger 5 is removed. In order to remove the blood, the pressure applied to the finger 5 is set to be not lower than maximum arterial blood pressure (about 200 mmHg) of the subject.

On the contrary, the pressure adjustment part 43 releases the air inside the bag 33 through the ventilation tube 34 to thereby reduce the volume of the air inside the bag 33. Thus, the bag 33 deflates to its original state shown in Fig. 1 so that the compression state of the subject's finger 5 can be released.

The signal output from the sensor 2 changes in accordance with the compression state and the compression release state. The information acquisition part 41 of the biological information acquisition apparatus 4 acquires biological information of the subject based on the change of the output signal. The information acquired thus is provided to a user such as a medical personnel through the information output part 42.

According to the aforementioned configuration, the bag 33 made of the soft material inflates to compress the subject's finger 5. Accordingly, uniform compression force can be applied non-locally. Accordingly, even if the relative position of the support tool 3 with respect to the subject's finger 5 changes whenever the support tool 3 is mounted, the influence of the change of the relative position on the compression state can be suppressed.

In addition, the sensor 2 is mounted on the subject's finger 5 outside the bag 33. Therefore, the relative position of the sensor 2 with respect to the subject's finger 5 does not change in accordance with inflation and deflation of the bag 33. Accordingly, even if the relative position of the support tool 3 with respect to the subject's finger 5 changes whenever the support tool 3 is mounted, the influence of the change of the relative position on a detection result made by the sensor 2 can be suppressed.

In addition, a pressurized state can be set or released by only sending air into the bag 33 or releasing air from the bag 33. Therefore, it is not necessary to build an actuator driving mechanism in the support tool 3. Accordingly, the size and weight of the support tool 3 can be reduced, and a burden imposed on the subject on whom the support tool 3 is mounted can be suppressed. Further, such a support tool 3 can be provided at low cost.

As described above, according to the aforementioned configuration, a specific compression state can be reproduced easily when biological information is acquired with compression of living tissues. In this manner, the biological information of the subject can be acquired quantitatively and reproductively.

The sensor 2 includes a light emitter 21. The light emitter 21 is mounted on the nail side 5a of the subject's finger 5. The light emitter 21 is configured to emit first light having a first wavelength $\lambda 1$ and second light having a second wavelength $\lambda 2$. The light emitter 21 includes a light emitting device which emits red light having a wavelength of 660 nm as an example of the first wavelength $\lambda 1$, and a light emitting device which emits infrared light having a wavelength of 940 nm as an example of the second wavelength $\lambda 2$. Each of the light emitting devices emits light at a predetermined timing in accordance with a control signal issued from the information acquisition part 41 of the biological information acquisition apparatus 4. For example, the light emitting device may be a light emission diode (LED) or a laser diode. The first light and the second light emitted thus are incident on the subject's finger 5 separately.

The sensor 2 includes a light receiver 22. The light receiver 22 is mounted on the ventral side 5b of the subject's finger 5. The light receiver 22 is disposed in a position where both the first light and the second light transmitted through the finger 5 can be received. The light receiver 22 is configured to output a first signal S1 corresponding to an intensity I1 of the received first light and a second signal S2 corresponding to an intensity I2 of the received second light. For example, an element functioning as the light receiver 22 may be a photodiode. The signals S1 and S2 output from the light receiver 22 are input to the information acquisition part 41 of the biological information acquisition apparatus 4.

The information acquisition part 41 may include a first light attenuation acquisition portion 411. The first light attenuation acquisition portion 411 is configured to acquire a light attenuation A1 of the first light based on the first signal S1 and acquire a light attenuation A2 of the second light based on the second signal S2. The light attenuations A1 and A2 are calculated respectively as ratios of quantities of received lights of the first signal S1 and the second signal S2 at a time instant (e.g. during compression of the living tissues) to quantities of received lights of the first signal S1 and the second signal S2 at another time instant (e.g. before compression of the living tissues). The light attenuations A1 and A2 are expressed by the following expressions respectively.

$$A1 = \log(I1/Io1) \qquad (1)$$

$$A2 = \log(I2/Io2) \qquad (2)$$

in which Io1, Io2 indicates the quantity of received light at a reference time instant (e.g. before compression of the living tissues); and I1, I2, the quantity of received light at a measurement time instant. A suffix "1" indicates the first light; and a suffix "2", the second light.

The information acquisition part 41 may include a second light attenuation acquisition portion 412. The second light attenuation acquisition portion 412 is configured to acquire a blood-based light attenuation Ab based on the light attenuations A1 and A2 of the first light and the second light acquired by the first light attenuation acquisition portion 411. Specifically, the second light attenuation acquisition portion 412 is configured to acquire the blood-based light attenuation Ab based on a difference between the light attenuation A1 and the light attenuation A2. The principle of this processing will be described below in detail.

When the bag 33 is inflated to compress the subject's finger 5, thicknesses of the living tissues change. Each of changes A of the light attenuations generated at that time is caused by a change in thickness of blood and a change in thickness of non-blood tissues. The fact can be expressed by the following expressions.

$$A1 = Ab1+At1 = E1HbDb+Z1Dt \qquad (3)$$

$$A2 = Ab2+At2 = E2HbDb+Z2Dt \qquad (4)$$

in which E indicates an extinction coefficient (dl g$^{-1}$ cm$^{-1}$); Hb, a hemoglobin concentration (g dl$^{-1}$) of the blood; Z, a light attenuation rate (cm$^{-1}$) of the non-blood tissues; and D, a changed thickness (cm). A suffix "b" indicates the blood; a suffix "t", the non-blood tissues; a suffix "1", the first light; and a suffix "2", the second light.

Since wavelength dependency of the non-blood tissues is ignorable, Z1 can be regarded as being equal to Z2. When the expression (3) is subtracted from the expression (4) here, the following expression is obtained.

$$A2\text{-}A1 = (E2\text{-}E1)HbDb$$

Only information about the blood is contained on the right side. Accordingly, when the difference between the light attenuation A1 and the light attenuation A2 is taken, the blood-based light attenuation Ab can be obtained.

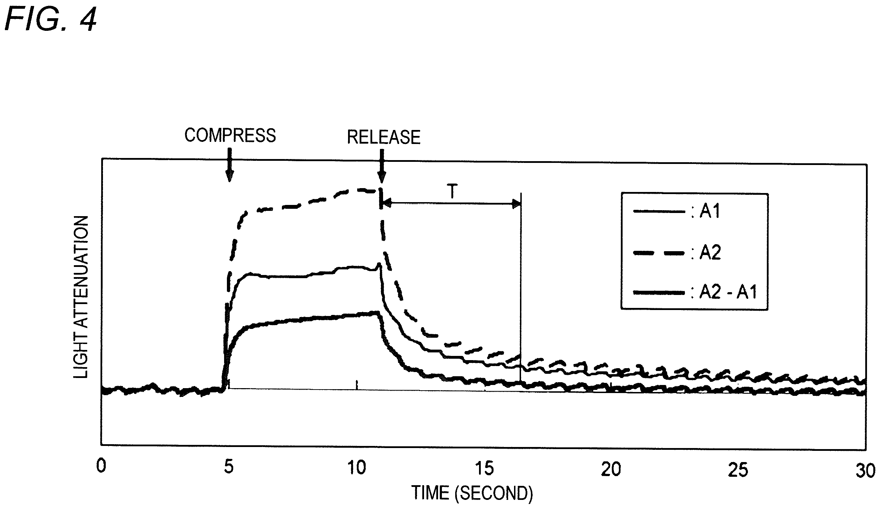

Fig. 4 is a graph showing temporal change of the light attenuation A1, the light attenuation A2 and the blood-based light attenuation Ab (=A2-A1) in the case where the finger 5 is compressed from above the sensor 2.

Even after the compression is released, the values of the light attenuations A1 and A2 do not return to levels prior to the start of the compression. Therefore, it is known that deformation of the non-blood tissues influences the values of the light attenuations A1 and A2. On the other hand, it is known that after the compression is released, the difference value (A2-A1) between the light attenuations, i.e. the blood-based light attenuation Ab converges at a level prior to the start of the compression. That is, the influence of the deformation of the non-blood tissues can be removed only by a simple arithmetic operation of taking a difference between the light attenuations obtained when the tissues are irradiated with the lights having the different wavelengths.

As shown in Fig. 1, the information acquisition part 41 may include a capillary refill time specifying portion 413. The capillary refill time specifying portion 413 is configured to specify a capillary refill time into the living tissues of the finger 5 based on a temporal change of the blood-based light attenuation Ab (= A2-A1) generated due to the compression of the subject's finger 5 by the bag 33 and obtained by the second light attenuation acquisition portion 412. Specifically, a proper threshold is determined so that, based on the threshold, it is possible to judge that the blood-based light attenuation Ab somewhat approximates to the level prior to the start of the compression. A time (T in Fig. 4) from a time point at which the compression is released to a time point at which the blood-based light attenuation Ab reaches the threshold is specified as the capillary refill time. Thus, the capillary refill time can be specified accurately without the influence of the deformation of the non-blood tissues caused by a difference in the degree of the compression.

The information acquisition part 41 inputs a signal ST to the information output part 42. The signal ST indicates the capillary refill time T specified by the capillary refill time specifying portion 413. The information output part 42 outputs the capillary refill time T in a suitable form corresponding to the signal ST. For example, the output form may be an indication based on at least one of a numerical value, a color and a symbol corresponding to the capillary refill time T, output of sound corresponding to the capillary refill time T, or the like.

According to the aforementioned configuration, the bag 33 can be inflated to reproduce the specific compression state of the subject's finger 5 easily. Accordingly, the capillary refill time of the subject can be measured quantitatively and reproducibly.

The light emitter 21 of the sensor 2 may be configured to emit third light having a third wavelength λ3 in addition to the first light having the first wavelength λ1 and the second light having the second wavelength λ2. The first wavelength λ1 and the second wavelength λ2 are wavelengths which are absorbed by hemoglobin, whereas the third wavelength λ3 is set as a wavelength at which absorption by water is larger than absorption by hemoglobin. For example, the third wavelength λ3 may be 1,300 nm. In this case, the light receiver 22 of the sensor 2 is configured to output a third signal S3 in accordance with an intensity of the third light transmitted through the subject's finger 5.

In the example, the information acquisition part 41 of the biological information acquisition apparatus 4 is configured to acquire information about the non-blood living tissues of the subject based on the third signal S3 output by the light receiver 22. Specifically, the first light attenuation acquisition portion 411 acquires a light attenuation A3 of the third light based on the third signal S3. The light attenuation A3 expresses information about light absorption by the blood and water of the non-blood living tissues. Therefore, when a value of the light attenuation A3 does not return to the level prior to the start of the compression even after the compression of the subject's finger 5 attained by the inflation of the bag 33 is released, presence of edema is suspected.

According to the aforementioned configuration, the bag 33 can be inflated to reproduce the specific compression state of the subject's finger 5 easily. Accordingly, diagnosis of edema can be performed reproducibly.

The information acquisition part 41 may be configured to acquire information about the non-blood living tissues of the subject also based on the first signal S1 and the second signal S2 in addition to the third signal S3. Specifically, with reference to the temporal change of the blood-based light attenuation Ab (= A2-A1) which has been acquired by the second light attenuation acquisition portion 412, it is possible to grasp to what extent the blood has returned to the living tissues after the compression preformed by the bag 33 is released. For example, assume that presence of edema has been confirmed based on the light attenuation A3. Even in this case, it is possible to estimate the edema to be mild when the return quantity of the blood is sufficient.

According to the aforementioned configuration, the bag 33 can be inflated to reproduce the specific compression state of the subject's finger 5 easily. Accordingly, the degree of edema can be performed quantitatively and reproducibly.

The light emitter 21 of the sensor 2 may be configured to emit light having N kinds of wavelengths that are different from one another, N being an integer equal to or greater than 3. For example, the light emitter 21 may be configured to emit first light having a first wavelength λ1, second light having a second wavelength λ, third light having a third wavelength λ3, and fourth light having a fourth wavelength λ4. In this case, the light emitter 21 includes a light emitting device which emits red light having a wavelength of 660 nm as an example of the first wavelength λ1, a light emitting device which emits infrared light having a wavelength of 940 nm as an example of the second wavelength λ2, a light emitting device which emits infrared light having a wavelength of 810 nm as an example of the third wavelength λ3, and a light emitting device which emits red light having a wavelength of 620 nm or 635 nm as an example of the fourth wavelength λ4. The respective light emitting devices emit the lights at predetermined timings in accordance with a control signal issued from the information acquisition part 41 of the biological information acquisition apparatus 4. For example, each of the light emitting devices may be a light emission diode (LED) or a laser diode. The first light, the second light, the third light and the fourth light emitted thus are incident on the subject's finger 5 separately.

In the example, the light receiver 22 is configured to output N kinds of signals in accordance with intensities of the N kinds of light transmitted through the subject's finger 5. Specifically, the light receiver 22 is disposed in a position where the first light, the second light, the third light and the fourth light transmitted through the finger 5 can be received. The light receiver 22 is configured to output a first signal S1 corresponding to an intensity I1 of the received first light, a second signal S2 corresponding to an intensity I2 of the received second light, a third signal S3 corresponding to an intensity I3 of the received third light, and a fourth signal S4 corresponding to an intensity I4 of the received fourth light. For example, an element functioning as the light receiver 22 may be a photodiode. The signals S1, S2, S3 and S4 output from the light receiver 22 are input to the information acquisition part 41 of the biological information acquisition apparatus 4.

In the example, the first light attenuation acquisition portion 411 is configured to acquire N kinds of light attenuations based on the N kinds of signals output by the light receiver 22. Specifically, the first light attenuation acquisition portion 411 is configured to acquire a first light attenuation A1 of the first light based on the first signal S1, acquire a second light attenuation A2 of the second light based on the second signal S2, acquire a third light attenuation A3 of the third light based on the third signal S3, and acquire a fourth light attenuation A4 of the fourth light based on the fourth signal S4. The light attenuations A1, A2, A3 and A4 are calculated respectively as ratios of quantities of received lights of the first signal S1, the second signal S2, the third signal S3 and the fourth signal S4 at a time instant (e.g. during compression of the living tissues) to quantities of received lights of the first signal S1, the second signal S2, the third signal S3 and the fourth signal S4 at another time instant (e.g. before compression of the living tissues). The light attenuations A1, A2, A3 and A4 are expressed by the following expressions respectively.

$$A1 = \log(I1/Io1) \quad (5)$$

$$A2 = \log(I2/Io2) \quad (6)$$

$$A3 = \log(I3/Io3) \quad (7)$$

$$A4 = \log(I4/Io4) \quad (8)$$

in which Io1, Io2, Io3, Io4 indicates the quantity of received light at a reference time instant (e.g. before compression of the living tissues); and I1, I2, I3, I4, the quantity of received light at a measurement time instant. A suffix "1" indicates the first light; a suffix "2", the second light; a suffix "3", the third light; and a suffix "4", the fourth light.

In this example, the second light attenuation acquisition portion 412 is configured to acquire at most N-1 kinds of blood-based light attenuations based on at most N-1 kinds of combinations of two light attenuations selected from the N kinds of light attenuations acquired by the first light attenuation acquisition portion 411. Specifically, the second light attenuation acquisition portion 412 is configured to acquire a blood-based light attenuation based on the light attenuations A1 and A2 of the first light and the second light acquired by the first light attenuation acquisition portion 411, a blood-based light attenuation based on the light attenuations A2 and A3 of the second light and the third light, and further a blood-based light attenuation based on the light attenuations A2 and A4 of the second light and the fourth light. More specifically, the second light attenuation acquisition portion 412 is configured to acquire a blood-based light attenuation Ab21 based on a different between the light attenuation A2 and the light attenuation A1, acquire a blood-based light attenuation Ab23 based on a different between the light attenuation A2 and the light attenuation A3, and acquire a blood-based light attenuation Ab24 based on a different between the light attenuation A2 and the light attenuation A4. The principle of this processing will be described below in detail.

When the bag 33 is inflated to compress the subject's finger 5, thicknesses of the living tissues change. Each of changes A of the light attenuations generated at that time is caused by a change in thickness of blood and a change in thickness of non-blood tissues. The fact can be expressed by the following expressions.

$$A1 = Ab1 + At1 = E1HbDb + Z1Dt \quad (9)$$

$$A2 = Ab2 + At2 = E2HbDb + Z2Dt \quad (10)$$

$$A3 = Ab3 + At3 = E3HbDb + Z3Dt \quad (11)$$

$$A4 = Ab4 + At4 = E4HbDb + Z4Dt \quad (12)$$

in which E expresses an extinction coefficient (dl g$^{-1}$ cm$^{-1}$), Hb, a hemoglobin concentration (g dl$^{-1}$) of the blood; Z, a light attenuation rate (cm$^{-1}$) of the non-blood tissues; and D, a thickness (cm). A suffix "b" expresses the blood; a suffix "t", the non-blood tissues; a suffix "1", the first light; a suffix "2", the second light; a suffix "3", the third light; and a suffix "4", the fourth light.

Since wavelength dependency of the non-blood tissues is ignorable, Z1, Z2, Z3 and Z4 can be regarded as being equal to one another. When the expression (9) is subtracted from the expression (10), the expression (11) is subtracted from the expression (10), and the expression (12) is subtracted from the expression (10) here, the following expressions are obtained.

$$Ab21 = A2 - A1 = (E2 - E1)HbDb \quad (13)$$

$$Ab23 = A2 - A3 = (E2 - E3)HbDb \quad (14)$$

$$Ab24 = A2 - A4 = (E2 - E4)HbDb \quad (15)$$

Only information about the blood is contained on the right side. Accordingly, when the difference between the light attenuation A2 and the light attenuation A1, the difference between the light attenuation A2 and the light attenuation A3, and the difference between the light attenuation A2 and the light attenuation A4 are taken, the blood-based light attenuations Ab21, Ab23 and Ab24 can be obtained.

Next, when the expression (13) is divided by the expression (14) and the expression (13) is divided by the expression (15), the items Hb and Db are removed, and the following expressions are obtained.

$$Ab21/Ab23 = (A2 - A1)/(A2 - A3) = (E2 - E1)/(E2 - E3) \quad (16)$$

$$Ab21/Ab24 = (A2 - A1)/(A2 - A4) = (E2 - E1)/(E2 - E4) \quad (17)$$

in which (E2-E1), (E2-E3) and (E2-E4) in the expression (16) and the expression (17) are functions of oxyhemoglobin O2Hb (%), deoxyhemoglobin RHb (%), and carboxyhemoglobin COHb (%). Extinction coefficients E1, E2, E3 and E4 are expressed by the following expressions respectively.

$$E1 = Eo1 \cdot O2Hb + Er1 \cdot RHb + Ec1 \cdot COHb \quad (18)$$

$$E2 = Eo2 \cdot O2Hb + Er2 \cdot RHb + Ec2 \cdot COHb \quad (19)$$

$$E3 = Eo3 \cdot O2Hb + Er3 \cdot RHb + Ec3 \cdot COHb \qquad (20)$$

$$E4 = Eo4 \cdot O2Hb + Er4 \cdot RHb + Ec4 \cdot COHb \qquad (21)$$

$$O2Hb + RHb + COHb = 1 \qquad (22)$$

in which Eo indicates an extinction coefficient of oxyhemoglobin; Er, an extinction coefficient of deoxyhemoglobin; and Ec, an extinction coefficient of carboxyhemoglobin. A suffix "1" indicates the first light; a suffix "2", the second light; a suffix "3", the third light; and a suffix "4", the fourth light. The reason why the differences between the light attenuations are taken is the same as previously described with reference to Fig. 4.

From the above description, it is know that when the lights having at least four wavelengths are used to measure the blood-based light attenuations Ab21, Ab23 and Ab24, an O2Hb concentration, an RHb concentration, and a COHb concentration in the blood can be specified quantitatively through the expressions (16) to (21).

As shown in Fig. 1, the information acquisition part 41 of the biological information acquisition apparatus 4 may include a blood substance concentration specifying portion 414. The blood substance concentration specifying portion 414 is configured to specify at most N-1 kinds of blood substance concentrations based on the at most N-1 kinds of blood-based light attenuations acquired by the second light attenuation acquisition portion 412. That is, the blood substance concentration specifying portion 414 is configured to specify the O2Hb concentration, the RHb concentration, and the COHb concentration based on the aforementioned principle. Also when COHb is replaced by MetHb, similar measurement can be made.

The information acquisition part 41 inputs a signal SC to the information output part 42. The signal SC indicates each of the blood substance concentrations (the O2Hb concentration, the RHb concentration, and the COHb concentration or the MetHB concentration) specified by the blood substance concentration specifying portion 414. The information output part 42 outputs the blood substance concentration in a suitable form corresponding to the signal SC. For example, the output form may be an indication based on at least one of a numerical value, a color and a symbol corresponding to the blood substance concentration, output of sound corresponding to the blood substance concentration, or the like.

When the number of wavelengths to be used is set at 5, four hemoglobin concentrations can be obtained. For example, an O2Hb concentration, an RHb concentration, a COHb concentration and an MetHb concentration can be obtained simultaneously. When one of 620 nm and 635 nm is set as the fourth wavelength λ4, the other of 620 nm and 635 nm can be used as an example of the fifth wavelength λ5.

When the number of wavelengths to be used is at least 3, the blood substance concentration specifying portion 414 can specify a blood oxygen saturation as an example of the blood substance concentration. The principle thereof will be described specifically.

(E2-E1) and (E2-E3) in the expression (16) are functions of the blood oxygen saturation S. Extinction Coefficients E1, E2, E3 will be expressed by the following expressions respectively.

$$E1 = Eo1S + Er1(1-S) \qquad (23)$$

$$E2 = Eo2S + Er2(1-S) \qquad (24)$$

$$E3 = Eo3S + Er3(1-S) \qquad (25)$$

in which Eo indicates an extinction coefficient of oxyhemoglobin; Er, an extinction coefficient of deoxyhemoglobin; and S, a blood oxygen saturation. Similarly to the above description, a suffix "1" indicates the first light; a suffix "2", the second light; and a suffix "3", the third light. Accordingly, a ratio between (E2-E1) and (E2-E3) is also a function of the blood oxygen saturation S.

From the above description, when the lights having at least three wavelengths are used to measure the blood-based light attenuations Ab21, Ab23 and Ab24, the blood oxygen saturation S can be specified quantitatively through the expression (16) and the expressions (23) to (25). The blood substance concentration specifying portion 414 may be configured to specify the blood oxygen saturation S based on this principle.

The expression "at most (N-1)" used in the aforementioned description intends to include the following case. In the case where, for example, the light emitter 21 is configured to emit lights having five wavelengths which are different from one another, it is not necessary to always obtain four blood substance concentrations. Configuration may be made so that the light emitter 21 configured thus is used to obtain three blood substance concentrations or less.

According to the aforementioned configuration, the bag 33 can be inflated to reproduce a specific compression state of the subject's finger 5 easily. Accordingly, the blood substance concentrations of the subject can be measured quantitatively and reproductively.

Here, the information output part 42 may be configured to output the blood substance concentrations in a state in which the subject's finger 5 is not compressed by the bag 33. Specifically, the information acquisition part 41 monitors the operation of the pressure adjustment part 43 for inflating or deflating the bag 33 to thereby grasp whether the subject's finger 5 is in a compression state or not. Since values of pulsation-borne blood substance concentrations cannot be specified in the compression state, the values can be specified only after the compression state is released. Accordingly, it is not preferable to present, to a user, the values of the blood substance concentrations in the compression state in real time. Therefore, the information output part 42 may be configured to suspend outputting the blood substance concentrations as long as it is determined by the information acquisition part 41 that the subject's finger 5 has been compressed by the bag 33. For example, the measurement values of the blood substance concentrations are prevented from being displayed in the biological information acquisition apparatus 4.

According to this configuration, effective measurement values can be always presented to the user while the blood substance concentrations of the subject can be measured quantitatively and reproducibly.

Alternatively, the information output part 42 may be configured to maintain the values output prior to the operation of the pressure adjustment part 43 for pressurizing the bag 33, as long as it is determined by the information acquisition part 41 that the subject's finger 5 has been compressed by the bag 33.

According to this configuration, the measurement values per se are constantly output. Therefore, effective measurement values can be always presented to the user with a feeling of security while the blood substance concentrations of the subject can be measured quantitatively and reproducibly.

As shown in Fig. 1, the biological information acquisition apparatus 4 may include a power supply 44. For example, the power supply 44 is a battery. The power supply 44 feeds electric power to the respective parts of the biological information acquisition apparatus 4 which require electric power for operating. In addition thereto or in place thereof, the power supply 44 may be configured to be capable of feeding electric power from an external commercial power supply to the respective parts of the biological information acquisition apparatus 4.

As shown in Fig. 1, the pressure adjustment part 43 may include a motor pump 431. The motor pump 431 is operated by electric power fed from the power supply 44. The motor pump 431 can perform a pressurization operation for sending air into the bag 33 through the ventilation tube 34, and a depressurization operation for removing the air inside the bag 33 through the ventilation tube 34.

The pressure adjustment part 43 controls the motor pump 431 to perform the pressurization operation to inflate the bag 33 to thereby compress the subject's finger 5. When, for example, the pressurization operation is performed for a fixed time, a compression state in which the finger 5 is compressed by the bag 33 can be formed. The pressure adjustment part 43 controls the motor pump 431 to perform the depressurization operation to deflate the bag 33. In this manner, the compression state can be released.

According to this configuration, the bag 33 can be inflated to compress the subject's finger 5 automatically, so that a specific compression state can be reproduced easily. In this manner, the biological information of the subject can be acquired quantitatively and reproducibly.

As shown in Fig. 1, the biological information acquisition apparatus 4 may include a pressure sensor 45. The pressure sensor 45 is configured to detect internal pressure of the bag 33. The pressure sensor 45 may directly detect the internal pressure of the bag 33, or may detect internal pressure of the ventilation channel (e.g. the inside of the ventilation tube 34) communicating the bag 33 and the pressure adjustment part 43 with each other to thereby indirectly detect the internal pressure of the bag 33.

In this case, the pressure adjustment part 43 may be configured to adjust the volume of air inside the bag so that the internal pressure of the bag 33 can reach a target value based on a detection result of the pressure sensor 45. For example, the pressure adjustment part 43 controls the motor pump 431 to continue the pressurization operation until the internal pressure of the bag 33 reaches the target value.

Even when the motor pump 431 performs a fixed operation, pressure applied to the subject's finger 5 may vary according to the shape of the subject's finger 5 or the mounting positions of the sensor 2 and the support tool 3. According to the aforementioned configuration, it is possible to grasp whether to what extent the pressure has been actually applied to the subject's finger 5. Therefore, a specific compression state can be reproduced easily regardless of the shape of the subject's finger 5 or the mounting positions of the sensor 2 and the support tool 3. In this manner, the biological information of the subject can be acquired more accurately and reproducibly.

As shown in Fig. 1, the support tool 3 may include a switch 35. The switch 35 is configured to be actuated when the subject's finger 5 is put into the support tool 3. For example, the switch 35 is provided in a space partitioned by the first support 31 and the second support 32. The switch 35 is configured to be actuated when the subject's finger 5 is disposed in a predetermined position in the space. For example, the switch 35 may be a mechanical switch, an optical sensor, a temperature sensor, etc.

In this case, the biological information acquisition apparatus 4 may include a control part 46. The control part 46 is configured to drive the motor pump 431 in accordance with the actuation of the switch 35.

According to this configuration, the pressurization operation can be started by the motor pump 431 automatically when the support tool 3 is mounted on the subject's finger 5. On the other hand, the pressurization operation can be prevented from being started by the motor pump 431 when the support tool 3 is not mounted in a proper position with respect to the subject's finger 5. Alternatively, the pressurization operation performed by the motor pump 431 can be suspended when the support tool 3 is displaced from the proper position with respect to the subject's finger 5 during measurement.

Accordingly, a specific compression state of the subject's finger 5 can be reproduced more easily. In this manner, the biological information of the subject can be acquired more accurately and reproducibly.

As shown in Fig. 1, the pressure adjustment part 43 may include a valve 432. The valve 432 is provided between the motor pump 431 and the ventilation tube 34. When the motor pump 431 is controlled to perform the pressurization operation with the valve 432 being closed, a state in which air is compressed can be formed on an upstream side of the valve 432. When the valve 432 is opened in this state, the compressed air flows into the bag 33 at high speed so that a pressurized state can be formed rapidly. On the contrary, when the motor pump 431 is controlled to perform the depressurization operation with the valve 432 being closed, a depressurized state can be formed on the upstream side of the valve 432. When the valve 432 is opened in this state, air inside the bag 33 can be released quickly. In this manner, delay of blood refill due to the residual pressure of the bag 33 can be suppressed. This configuration is particularly useful in the case where a small-sized motor pump 431 having relatively low air-sending capability is used.

The control part 46 may be configured to operate the pressure adjustment part 43 at a predetermined interval. The interval may be variable suitably in accordance with a usage mode of the biological information acquisition apparatus 4.

According to this configuration, the bag 33 can be inflated to compress the subject's finger 5 automatically whenever a predetermined time elapses. A specific compression state can be reproduced easily on this occasion. Therefore, for example, patient's therapy effect or deteriorating tendency of patient's condition during an operation or in an intensive care unit (ICU) can be checked automatically and accurately.

As shown in Fig. 1, the biological information acquisition apparatus 4 may include a switch 47. The switch 47 is configured to start the operation of the pressure adjustment part 43. For example, the switch 47 is a measurement start switch which is provided in an operating panel of the biological information acquisition apparatus 4.

In this case, the control part 46 (an example of a prohibition control part) may be configured to invalidate the operation of the switch 47 unless a predetermined time elapses after compression of the subject's finger 5 by the bag 33 is released. For example, the predetermined time is set as a time which is required until blood removed from the living tissues of the finger 5 due to the compression given by the bag 33 sufficiently returns to the living tissues due to the release of the compression.

After the compression of the subject's finger 5 by the bag 33 is released, a certain amount of time is required until the blood returns to the living tissues of the finger 5 to restore the finger 5 to its original state. When the switch 47 is operated prior to the restoration, biological information of the subject obtained through compression performed based on the operation may lack accuracy. According to the aforementioned configuration, next measurement can be prevented from being carried out before the subject's finger 5 which has been released from the compression is restored to its original state. Accordingly, the biological information of the subject can be acquired more accurately and reproducibly while a specific compression state of the subject's finger 5 can be reproduced easily.

The control part 46 may be configured to be capable of changing the aforementioned predetermined time in accordance with the operation state of the pressure adjustment part 43. For example, assume that the aforementioned predetermined time is normally set as thirty seconds. Even in this case, setting can be made to invalidate the operation of the switch 47 for five minutes after compression has been performed five times within three minutes.

When the compression is performed by the bag 33 repeatedly, a burden imposed on the living tissues of the subject's finger 5 becomes large in spite of non-local pressure. According to the aforementioned configuration, the burden imposed on the subject can be suppressed while a specific compression state of the subject's finger 5 can be reproduced easily.

As shown in Fig. 1, the biological information acquisition apparatus 4 may include a power supply monitoring part 48. The power supply monitoring part 48 is configured to monitor power feeding performance from the power supply 44 to the motor pump 431. The power supply monitoring part 48 is configured to control the pressure adjustment part 43 to stop driving the motor pump 431 when the power feeding performance is lower than a predetermined value. For example, the circumstance that the power feeding capacity is lower than the predetermined value may be a case where the residual level of a battery is lower than the predetermined value, a case where a blackout occurred while the commercial power supply is in use, or the like.

When the motor pump 431 is driven under a circumstance that power is fed insufficiently, a desired compression state of the subject's finger 5 may not be able to be obtained. According to the aforementioned configuration, a situation that measurement is made in this state to acquire inaccurate biological information of the subject can be avoided. Accordingly, the biological information of the subject can be acquired more accurately and reproducibly while a specific compression state of the subject's finger 5 can be reproduced easily.

As shown in Fig. 1, the pressure adjustment part 43 of the biological information acquisition apparatus 4 may include a manual adjustment mechanism 433 in addition to or in place of the motor pump 431. The manual adjustment mechanism 433 is a mechanism configured to be capable of manually adjusting the volume of air inside the bag 33. For example, the manual adjustment mechanism 433 includes a check valve and an air-sending piece. The check valve may be provided in the middle of the ventilation tube 34. For example, the air-sending piece may be a rubber ball, a syringe, etc. When the air-sending piece is operated, air is sent into the bag 33 through the ventilation tube 34. Thus, the bag 33 inflates. In addition, deflation can be prevented by the check valve. When the compression state generated by the bag 33 has to be released, it will go well as long as the check valve is opened.

In the case of the configuration in which the manual adjustment mechanism 433 is provided in place of the motor pump 431, the configuration of the pressure adjustment part 43 can be simplified, and the size and weight of the biological information acquisition apparatus 4 can be reduced. In the case of the configuration in which the manual adjustment mechanism 433 is provided in addition to the motor pump 431, biological information can be acquired manually even when the power feeding performance of the power supply 44 to the motor pump 431 becomes insufficient. When the power supply 44 is a battery, biological information can be acquired manually if necessary. As a result, electric power can be saved. Accordingly, the biological information of the subject can be measured in accordance with the circumstance while a specific compression state of the subject's finger 5 can be reproduced easily.

As shown in Fig. 1, the biological information acquisition apparatus 4 may include an audio output part 49. The audio output part 49 is configured to output sound corresponding to the pressure applied to the subject's finger 5. Since the pressure corresponds to the internal pressure of the bag 33, the audio output part 49 can use a detection result of the aforementioned pressure sensor 45. Alternatively, configuration may be made so that a pressure sensor which can more directly detect the pressure applied to the subject's finger 5 is provided separately in the support tool 3, and the audio output part 49 uses the detection result of the pressure sensor. For example, the audio output part 49 may output predetermined sound when the pressure applied to the finger 5 reaches a predetermined value. Alternatively, the audio output part 49 may output sound so that the sound volume or interval can change as the pressure applied to the finger 5 increases.

According to this configuration, the user can easily grasp whether the subject's finger 5 is in a compression state or not. Particularly in the case where pressure is given by the bag 33 manually, the user can use the sound output by the audio output part 49, as a guide for forming a desired compression state. Accordingly, the biological information of the subject can be acquired accurately and reproducibly while a specific compression state of the subject's finger 5 can be reproduced more easily.

In each of the aforementioned configurations, the light emitter 21 of the sensor 2 is mounted on the nail side 5a of the subject's finger 5, and the light receiver 22 of the sensor 2 is mounted on the ventral side 5b of the finger 5. However, the light emitter 21 may be mounted on the ventral side 5b of the finger 5 and the light receiver 22 may be mounted on the nail side 5a of the finger 5.

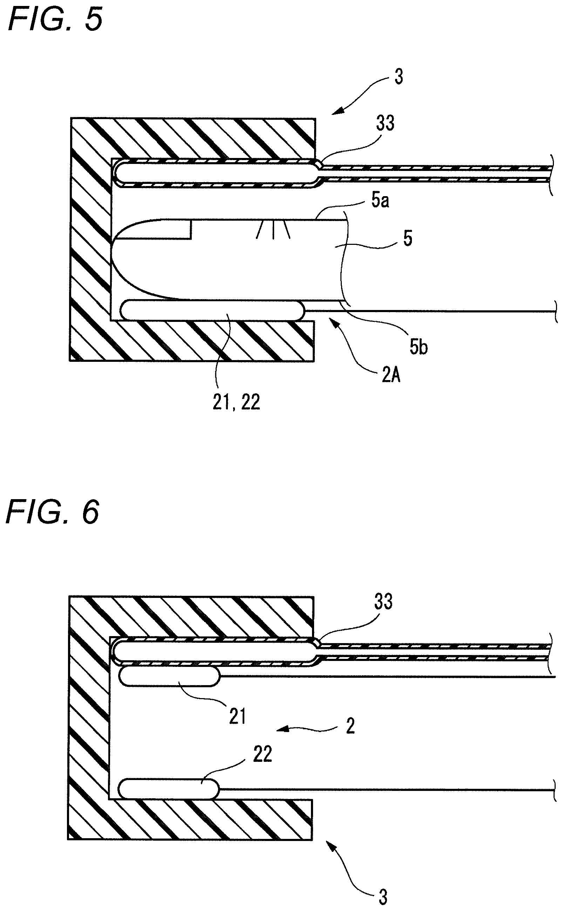

In each of the aforementioned configurations, light emitted from the light emitter 21 and transmitted through the subject's finger 5 is incident on the light receiver 22. However, an optical sensor 2A may be used alternatively, as shown in Fig. 5. In the optical sensor 2A, both a light emitter 21 and a light receiver 22 are mounted on the same side of the subject's finger 5, and light emitted from the light emitter 21 and reflected on the subject's finger 5 is incident on the light receiver 22. In the example of Fig. 5, the optical sensor 2A is mounted on the ventral side 5b of the finger 5. However, the optical sensor 2A may be mounted on the nail side 5a of the finger 5.

In each of the aforementioned configurations, the support tool 3 is mounted on the finger 5 of the subject on which the sensor 2 is mounted. However, as shown in Fig. 6, the support tool 3 may be configured such that the sensor 2 is attached to the support tool 3 in advance. The light emitter 21 and the reception part 22 of the sensor 2 are disposed at at least one of between the subject's finger 5 and the bag 33 and between the subject's finger 5 and the second support 32. In the example of Fig. 6, the light emitter 21 is disposed on an outer face of the bag 33 and the light receiver 22 is disposed on an inner peripheral face of the second support 32.

According to this configuration, biological information can be acquired by only an operation of mounting the support tool 3 on the subject's finger 5. Accordingly, the biological information of the subject can be acquired simply and reproducibly while a specific compression state of the subject's finger 5 can be reproduced easily.

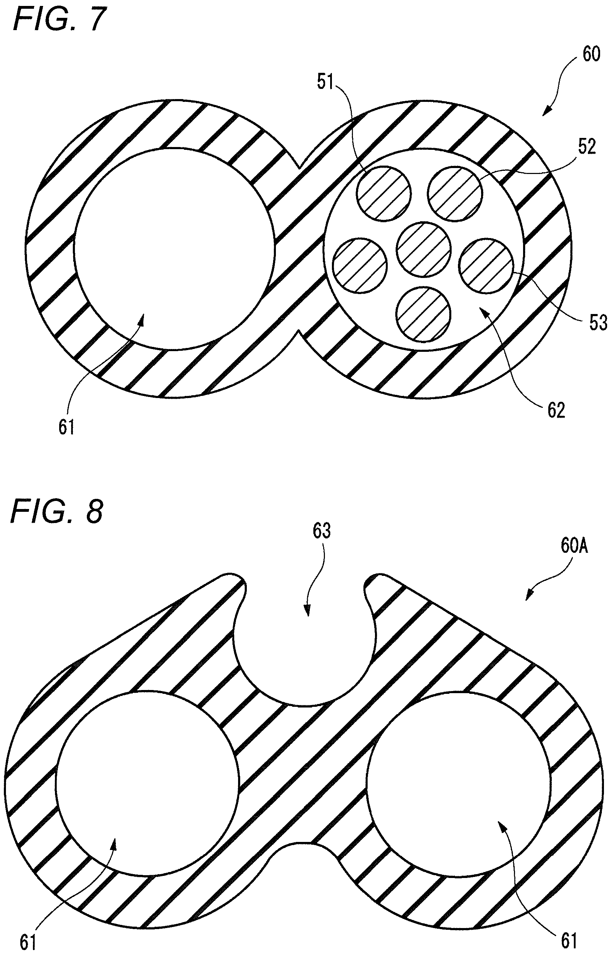

As shown in Fig. 1, the light emitter 21 of the sensor 2 is connected to the information acquisition part 41 of the biological information acquisition apparatus 4 through a signal line 51. The light mission part 21 of the sensor 2 is connected to the information acquisition part 41 of the biological information acquisition apparatus 4 through a signal line 52. When the support tool 3 includes the switch 35, the switch 35 is connected to the control part 46 of the biological information acquisition apparatus 4 through a signal line 53. The support apparatus 3 and the biological information acquisition apparatus 4 may be connected by a tube 60 higher in softness than the first support 31 and the second support 32 of the support tool 3. A configuration example of such a tube 60 will be shown in Fig. 7.

Fig. 7 shows a section taken along a direction perpendicular to an extension direction of the tube 60. The tube 60 includes a ventilation channel 61 and a signal line housing part 62. The ventilation channel 61 is a part which functions as the ventilation tube 34 and through which the pressure adjustment part 43 of the biological information acquisition apparatus 4 and the bag 33 of the support tool 3 communicate with each other. The signal line housing part 62 is a part which houses the signal lines 51, 52, 53.

Alternatively, the support apparatus 3 and the biological information acquisition apparatus 4 may be connected to each other by a tube 60A shown in Fig. 8. Fig. 8 shows a section taken along a direction perpendicular to an extension direction of the tube 60A. The tube 60A includes a pair of ventilation channels 61 and a signal line retention part 63. The ventilation channels 61 are parts which may function as the ventilation tube 34 and through which the pressure adjustment part 43 of the biological information acquisition apparatus 4 and the bag 33 of the support tool 3 communicate with each other. Configuration may be made so that only one of the ventilation channels 61 is provided. The signal line retention part 63 is a part which retains the signal lines 51, 52, 53 in a removable manner.

When the tube 60 or the tube 60A is used, an electric system cable and a ventilation system hose can be combined into a single line. In this manner, dislocation of the support tool 3 due to body motion etc. of the subject can be suppressed, in comparison with a case where two lines may be displaced individually due to the body motion etc. Accordingly, a specific compression state of the subject's finger 5 can be reproduced more easily. Thus, the biological information of the subject can be acquired more reproducibly.

In the aforementioned configuration, the pressure adjustment part 43 of the biological information acquisition apparatus 4 adjusts the volume of air inside the bag 33 through the ventilation tube 34. However, a suitable fluid may be used as long as it can inflate or deflate the bag 33. In this case, the ventilation tube 34 has a configuration in which the fluid can be circulated through the ventilation tube 34.

Next, a specific configuration of the support tool 3 belonging to the biological information acquisition system 1 configured in the aforementioned manner will be described. In the following description, the terms "front", "rear", "left", "right", "up" and "down" are used for the convenience of illustration, and do not limit an orientation or direction in an actual usage state.

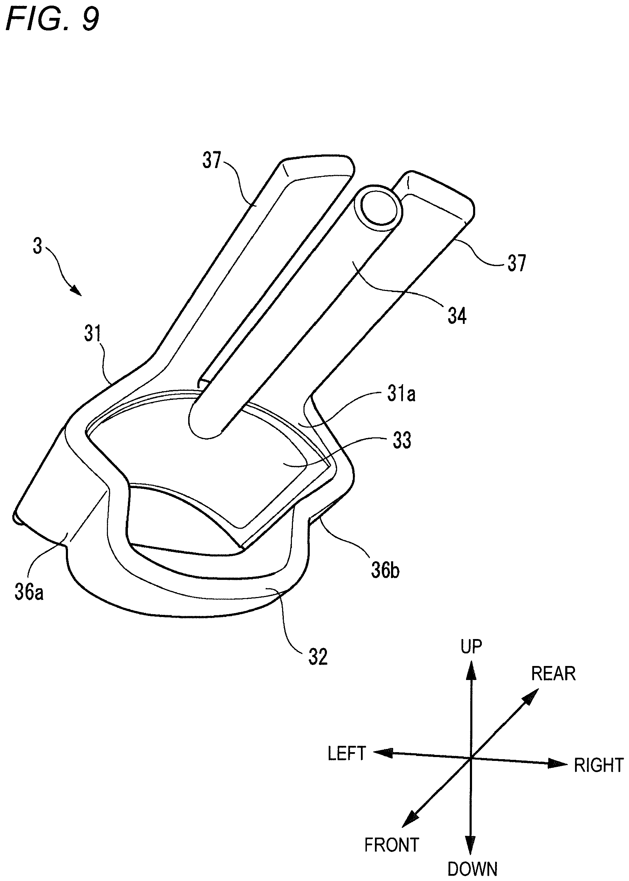

Fig. 9 shows a support tool 3 according a first embodiment. In the support tool 3, a left end potion of a first support 31 and a left end portion of a second support 32 are connected by a connection wall 36a, and a right end potion of the first support 31 and a right end portion of the second support 32 are connected by a connection wall 36b. When the support tool 3 is mounted onto a finger 5 of a subject, the finger 5 is surrounded by the first support 31, the second support 32 and the connection walls 36a and 36b.

An inner peripheral face 31 a of the first support 31 extends in the shape of an arc (the shape of an arc). A bag 33 is disposed to extend along the inner peripheral face 31a. One end of a ventilation tube 34 (only a part thereof is illustrated) is molded integrally with the bag 33. The bag 33 which inflates due to air sent thereto from the pressure adjustment part 43 of the biological information acquisition apparatus 4 through the ventilation tube 34 abuts against a peripheral part of the subject's finger 5 to thereby apply compression thereto.

According to this configuration, as shown in Fig. 3, a pressurized state can be formed by a smaller volume change of the bag 33, in comparison with a configuration in which a bag is mounted on a flat inner peripheral face. In other words, the pressurized state can be formed by use of the bag 33 having a smaller volume. That is, the pressurized state can be formed rapidly even by use of a smaller-sized air-sending mechanism. A similar effect can be obtained also when the bag 33 is deflated to release the pressurized state. Accordingly, the size of the apparatus can be reduced or cost can be suppressed while a specific compression state of the subject's finger 5 can be reproduced easily.

As shown in Fig. 9, the support tool 3 includes fixation assistance parts 37. Each of the fixation assistance parts 37 extends rearward from a rear end portion of the first support 31. The fixation assistance part 37 is provided to extend along the finger 5 in order to assist fixation of the support tool 3 to the subject's finger 5.

According to this configuration, a user can attach the support tool 3 on the subject's finger 5 with the fixation assistance parts 37 as a guide. When the fixation assistance parts 37 are fixed to the finger 5 by a tape etc. if necessary, dislocation of the support tool 3 with respect to the finger 5 during measurement can be prevented. Accordingly, a specific compression state of the finger 5 can be reproduced more easily. Thus, biological information of the subject can be acquired more reproducibly.

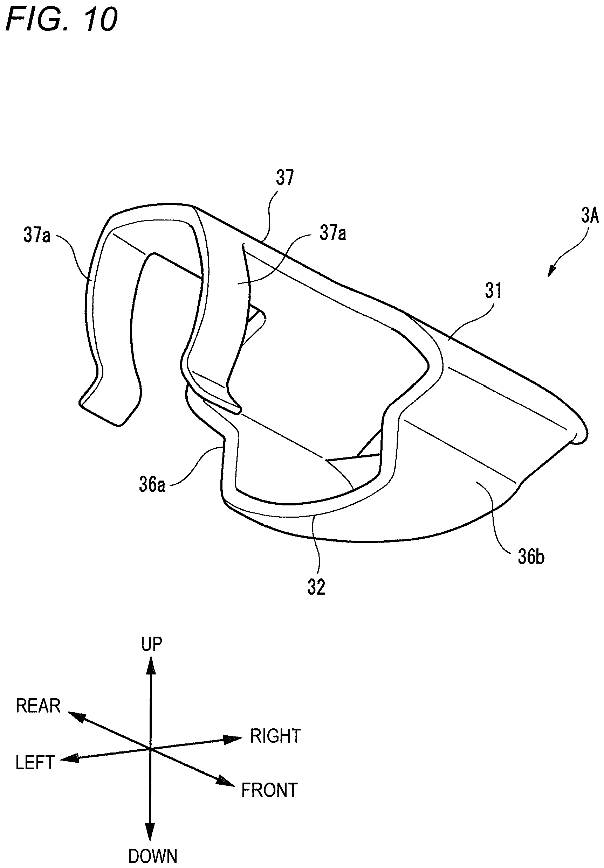

Fig. 10 shows a support tool 3A according to a second embodiment. Elements having identical or equivalent configurations or functions to those of the support tool 3 according to the first embodiment will be referred to by the same numerals correspondingly and respectively, and duplicate description thereof will be omitted. In Fig. 10, a bag 33 and a ventilation tube 34 are omitted from illustration.

In the support tool 3A, a pair of elastic pieces 37a (an example of elastic members) are provided on rear end portions of a fixation assistance piece 37. When the support tool 3A is mounted on a finger 5 of a subject, the elastic pieces 37a hold a part of the finger 5 to thereby surround the part of the finger 5.

According to this configuration, the support tool 3A can be fixed to the subject's finger 5 without requiring an auxiliary fixation piece such as a tape. In this manner, dislocation of the support tool 3 with respect to the finger 5 during measurement can be prevented, so that a specific compression state of the finger 5 can be reproduced more easily. Accordingly, biological information of the subject can be acquired more reproducibly.

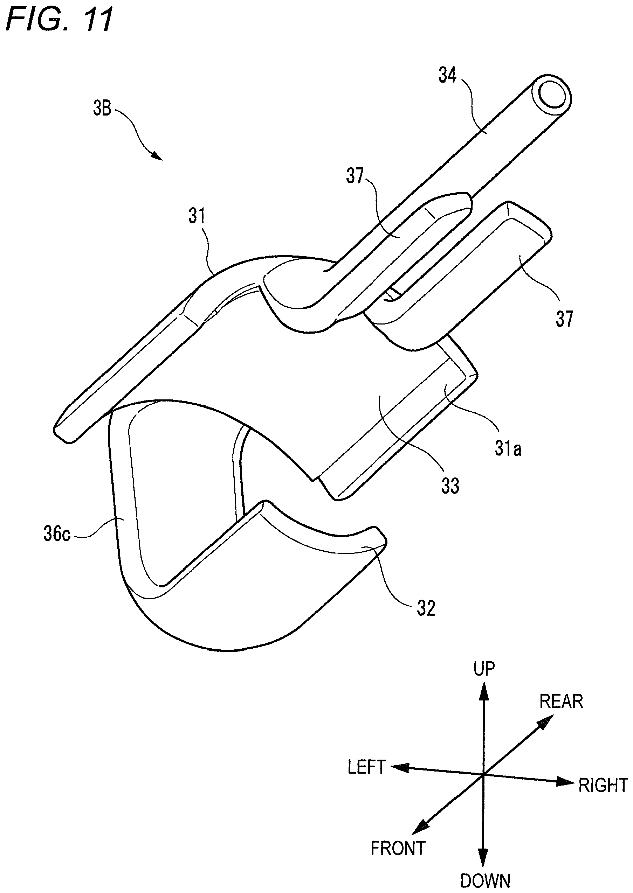

Fig. 11 shows a support tool 3B according to a third embodiment. Elements having identical or equivalent configurations or functions to those of the support tool 3 according to the first embodiment will be referred to by the same numerals correspondingly and respectively, and duplicate description thereof will be omitted.

In the support tool 3B, a front end portion of a first support 31 and a front end portion of a second support 32 are connected by a connection wall 36c. When the support tool 3B is mounted on a finger 5 of a subject, the finger 5 is disposed in a space partitioned by the first support 31, the second support 32 and the connection wall 36c. The space is opened in a left/right direction.

That is, the whole periphery of the subject's finger 5 does not have to be surrounded during acquisition of biological information. When a portion having relatively high rigidity is set to the necessary minimum, an increase in the weight of the support tool 3B can be suppressed. Accordingly, a burden imposed on the subject on whom the support tool 3B is mounted can be suppressed.

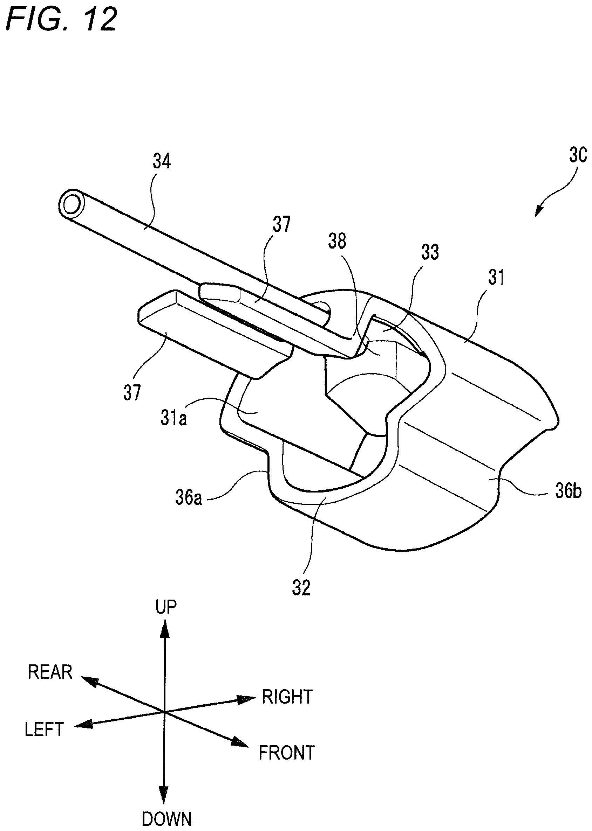

Fig. 12 shows a support tool 3C according to a fourth embodiment. Elements having identical or equivalent configurations or functions to those of the support tool 3 according to the first embodiment will be referred to by the same numerals correspondingly and respectively, and duplicate description thereof will be omitted.

The support tool 3C includes a pressing member 38. The pressing member 38 is a member that resembles a finger of a living body. Specifically, the pressing member 38 is made of a soft material that resembles a finger of a living body. The pressing member 38 has a shape that ressembles a finger which has been compressed by a hand of a medical personnel. The pressing member 38 is provided on an outer face of a bag 33. When the bag 33 inflates due to air sent thereto from the pressure adjustment part 43 of the biological information acquisition apparatus 4 through a ventilation tube 34, the pressing member 38 abuts against a finger 5 of a subject.

According to this configuration, it is possible to form a more natural compression state which is similar to the compression heretofore performed by a hand of a medical personnel. Accordingly, biological information can be acquired more naturally while a specific compression state of the subject's finger 5 can be reproduced easily.

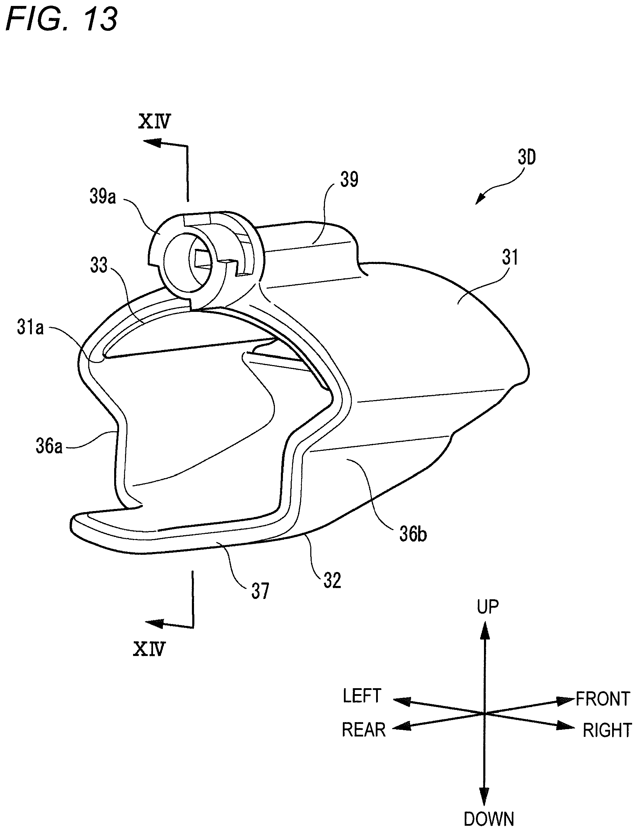

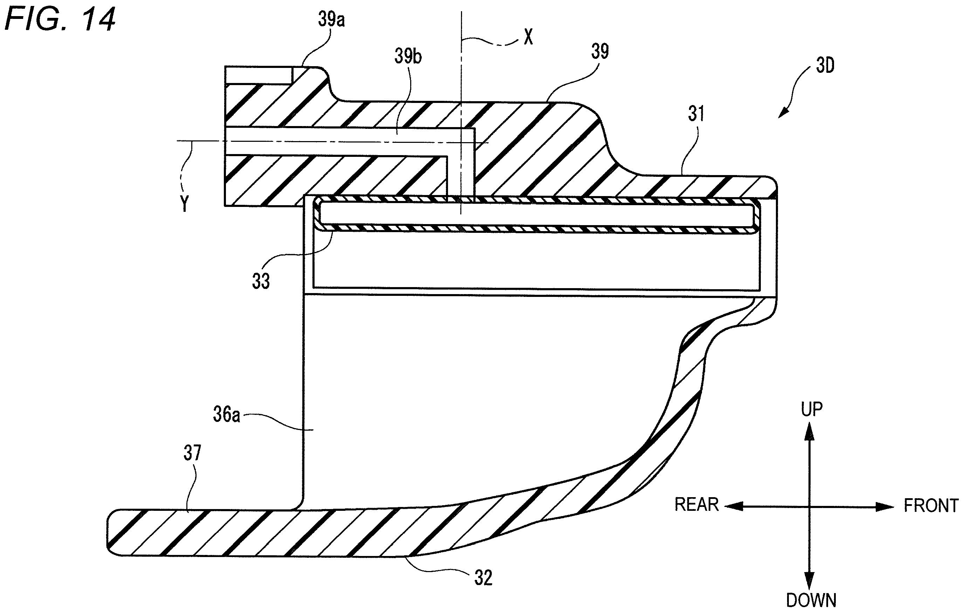

Fig. 13 and Fig. 14 show a support tool 3D according to a fifth embodiment. Fig. 14 is a longitudinal sectional view taken along a line XIV-XIV in Fig. 13. Elements having identical or equivalent configurations or functions to those of the support tool 3 according to the first embodiment will be referred to by the same numerals correspondingly and respectively, and duplicate description thereof will be omitted.

As shown in Fig. 13, the support tool 3D has a fixation assistance part 37. The fixation assistance part 37 is provided on a rear end portion of a second support 32 and extends rearward. In addition, the support tool 3D includes a channel formation part 39. The channel formation part 39 is provided on an outer peripheral face of a first support 31. The channel formation part 39 has a connection portion 39a. As shown in Fig. 14, a ventilation channel 39b is formed inside the channel formation part 39. One end of the ventilation channel 39b is opened in the connection portion 39a. The other end of the ventilation channel 39b communicates with the inside of the bag 33 mounted on an inner peripheral face 31 a of the first support 31. When one end of a not-shown ventilation tube 34 is connected to the connection portion 39a, a ventilation channel ranging from the pressure adjustment part 43 of the biological information acquisition apparatus 4 to the bag 33 is formed.

According to this configuration, the support tool 3D can be attached to the ventilation tube 34 removably. Thus, the ventilation tube 34 does not have to be molded integrally with the bag 33 so that manufacturing cost of the support tool 3D can be suppressed. Accordingly, it is possible to meet a request to make only the support tool 3D disposable while reproducing a specific compression state of the subject's finger 5 easily.

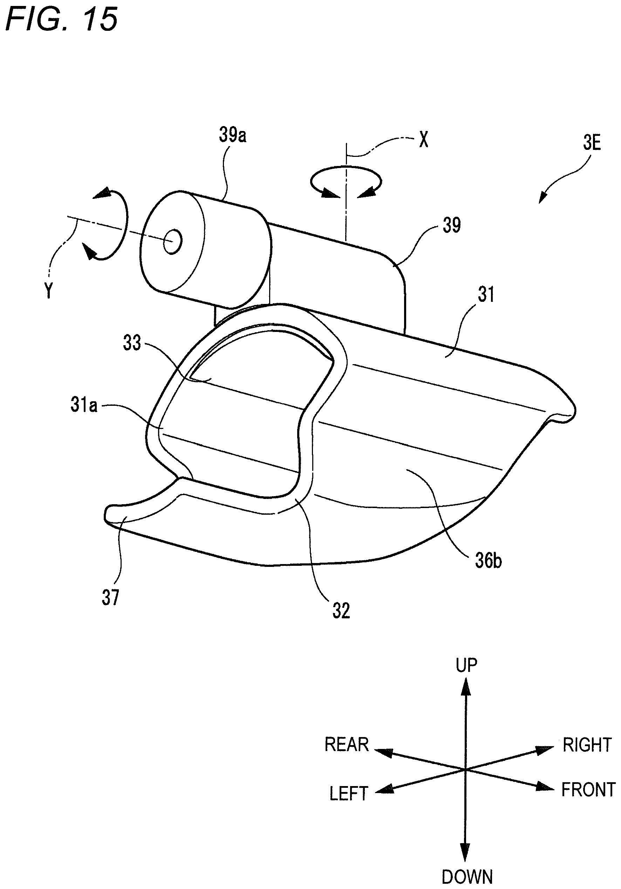

Fig. 15 shows a support tool 3E according to a sixth embodiment. Elements having identical or equivalent configurations or functions to those of the support tool 3D according to the fifth embodiment will be referred to by the same numerals correspondingly and respectively, and duplicate description thereof will be omitted.

In the support tool 3E, a channel formation part 39 is made rotatable around a central axis X within a plane perpendicular to the central axis X. The central axis X extends in an up/down direction of the support tool 3E. The central axis X extends along a portion where a ventilation channel 39b extends in the up/down direction of the support tool 3E (see Fig. 14). Thus, the channel formation part 39 is made movable relatively to a first support 31.

In the support tool 3E, a connection portion 39a is made rotatable around a central axis Y within a plane perpendicular to the central axis Y. The central axis Y extends in a front/rear direction of the support tool 3E. The central axis Y extends along a portion where the ventilation channel 39b extends in the front/rear direction of the support tool 3E (see Fig. 14). Thus, the connection portion 39a is made movable relatively to the first support 31.

According to this configuration, even when a ventilation tube 34 connected to the connection portion 39a is displaced due to body motion of a subject, the channel formation part 39 is displaced following the displacement of the ventilation tube 34. Accordingly, dislocation of the support tool E due to the body motion can be suppressed. Accordingly, a specific compression state of a finger 5 of the subject can be reproduced more easily. Thus, biological information of the subject can be acquired more reproducibly.

The channel formation part 39 and the connection portion 39a may be configured such that one of them is movable relative to the first support 31.

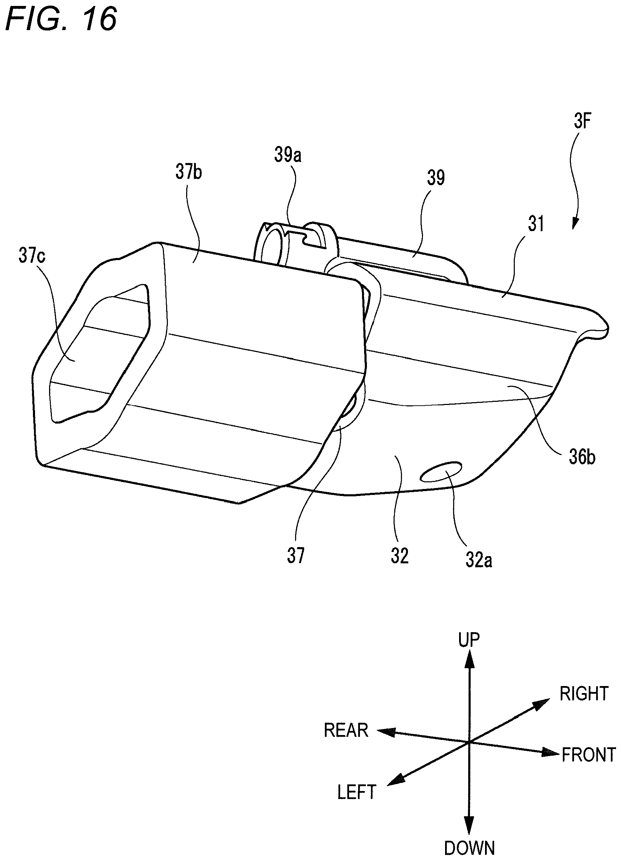

Fig. 16 shows a support tool 3F according to a seventh embodiment. Elements having identical or equivalent configurations or functions to those of the support tool 3D according to the fifth embodiment will be referred to by the same numerals correspondingly and respectively, and duplicate description thereof will be omitted.

The support tool 3F includes an elastic member 37b on a rear end portion of a fixation assistance part 37. A through hole 37c extending in a front/rear direction of the support tool 3F is formed in the elastic member 37b. The elastic member 37b is made of a material which has elasticity and which is higher in softness than the fixation assistance part 37.

To attach the support tool 3F on a finger 5 of a subject, the finger 5 is first inserted into the through hole 37c of the elastic member 37b. The finger 5 passes through the elastic member 37b while enlarging the through hole 37c. As a result, the finger 5 is disposed in a predetermined position opposed to a bag 33. The elastic member 37b retains the finger 5 due to its own elasticity while surrounding a part of the finger 5.

According to this configuration, the support tool 3F can be fixed to the subject's finger 5 without requiring an auxiliary fixation piece such as a tape. Thus, dislocation of the support tool 3 with respect to the finger 5 during measurement can be prevented, so that a specific compression state of the finger 5 can be reproduced more easily. Accordingly, biological information of the subject can be acquired more reproducibly.

In the support tool 3F, a through hole 32a is formed in a second support 32. The through hole 32a communicates with a space where the subject's finger 5 is disposed. The position, shape and size of the through holes 32a are determined to allow access to the finger 5 of the subject on whom the support tool 3F is mounted. The access may be made by a rod-like jig or a user's finger.

According to this configuration, even in an emergency that the pressure adjustment part 43 of the biological information acquisition apparatus 4 cannot function for some reason, the subject's finger 5 can be compressed by a user's hand so that biological information can be acquired.

The through hole 32a may be formed in a first support 31. In this case, the bag 33 is pressed against the subject's finger 5 by a rod-like jig or a user's finger.

Features described about each of the aforementioned embodiments may be combined suitably with configurations of other embodiments as long as the original configurations or functions of the other embodiments are not spoiled.

The above description is for facilitating the understanding of the present invention, and does not limit the present invention. The present invention may be changed or modified without departing from the gist of the invention and it is apparent that their equivalents are also included in the invention.

The biological information acquisition apparatus 4 according to the aforementioned embodiment acquires a capillary refill time and blood substance concentrations of a subject as biological information. In other words, the support tool 3 (3A to 3F) according to each of the aforementioned embodiments is mounted on a finger 5 of the subject to support acquisition of the capillary refill time and the blood substance concentrations of the subject. However, the support tool 3 (3A to 3F) according to the invention can be applied to support of acquisition of all biological information which can be acquired by use of the sensor of a type which can be mounted on the subject's finger 5. For example, the biological information may be a heart rate, body temperature, etc.

In the support tool 3 (3A to 3F) according to each of the aforementioned embodiments, the bag 33 is disposed along the inner peripheral face 31a of the first support 31 disposed on the nail side 5a of the finger. However, the support tool 3 (3A to 3F) according to each of the aforementioned embodiments may have a configuration in which the first support 31 is disposed on the ventral side 5b of the finger 5. In this case, the bag 33 is disposed on the ventral side 5b of the finger 5, and the second support 32 is disposed on the nail side 5a of the finger 5. In addition, the support tool 3 (3A to 3F) according to each of the aforementioned embodiments may have a configuration in which the first support 31 is disposed on one of the left and right sides of the finger 5. In this case, the bag 33 is disposed on the one of the left and right sides of the finger 5, and the second support 32 is disposed on an opposite side to the first support 31 with the interposition of the bag 33 therebetween.

In the support tool 3 (3A to 3F) according to each of the aforementioned embodiments, the whole of the bag 33 is disposed along the inner peripheral face 31a of the first support 31. However, the support tool 3 (3A to 3F) according to each of the aforementioned embodiments may have a configuration in which at least a part of the bag 33 is disposed along the inner peripheral face 31a of the first support 31. In the case where, for example, the first support 31 is disposed on the nail side 5a of the subject's finger 5, a part of the bag 33 may be formed into a shape going around to the ventral side 5b of the finger 5.

In the embodiments illustrated so far, the functions of the information acquisition part 41, the control part 46, the power supply monitoring part 48, the first light attenuation acquisition portion 411, the second light attenuation acquisition portion 412, the capillary refill time specifying portion 413, and the blood substance concentration specifying portion 414 are achieved by software executed by cooperation between a processor and a memory which are connected communicably. For example, the processor may be a CPU or an MPU. For example, the memory may be an RAM or an ROM. However, at least one of the functions of the information acquisition part 41, the control part 46, the power supply monitoring part 48, the first light attenuation acquisition portion 411, the second light attenuation acquisition portion 412, the capillary refill time specifying portion 413, and the blood substance concentration specifying portion 414 may be achieved by hardware such as a circuit element or by a combination of hardware and software.

The content of Japanese Patent Application No. 2014-047755 filed on March 11, 2014 is incorporated herein as forming a part of the description of the present application.

**Claims**

1. A support tool to be mounted on a finger of a subject on which a sensor is mounted so as to support an acquisition of biological information of the subject, the support tool comprising:

   a first support to be disposed, when mounted, on a first side of the finger of the subject, the first support comprising an inner peripheral face;
   a second support to be disposed, when mounted, on a second side of the finger of the subject opposite to the first side, a relative position of the second support with respect to the first support being fixed;
   a bag made of a material softer than the first support and the second support, the bag being at least partially disposed along the inner peripheral face; and
   a channel formation part forming a fluid channel communicating with an inside of the bag,

   wherein the support tool is configured such that, when mounted, the sensor is disposed at at least one of between the finger of the subject and the bag and between the finger of the subject and the second support.

2. The support tool according to claim 1, wherein the inner peripheral face extends in an arc shape.

3. The support tool according to claim 1 or 2, wherein a pressing member that resembles a finger of a living body is provided on an outer face of the bag.

4. The support tool according to any one of claims 1 to 3, wherein a fixation assistance part is provided on at least one of the first support and the second support to assist a fixation to the finger of the subject.

5. The support tool according to claim 4, wherein the fixation assistance part comprises an elastic member that surrounds a part of the finger of the subject.

6. The support tool according to any one of claims 1 to 5, wherein the channel formation part is provided on an outer peripheral face of the first support, the channel formation part comprising a connection portion to be connected to a tube communicating with the fluid channel.

7. The support tool according to claim 6, wherein at least one of the channel formation part and the connection portion is movable relative to the first support.

8. The support tool according to any one of claims 1 to 7, wherein a through hole allowing an access to the finger of the subject is formed through the first support or the second support.

9. The support tool according to any one of claims 1 to 8, wherein the biological information includes at least one of a capillary refill time and a blood substance concentration.

10. A support tool to be mounted on a finger of a subject to support an acquisition of biological information of the subject, the support tool comprising
    a first support to be disposed, when mounted, on a first side of the finger of the subject, the first support comprising an inner peripheral face;
    a second support to be disposed, when mounted, on a second side of the finger of the subject opposite to the first side, a relative position of the second support with respect to the first support being fixed;

a bag made of a material softer than the first support and the second support, the bag being at least partially disposed along the inner peripheral face;

a channel formation part forming a fluid channel communicating with an inside of the bag; and

a sensor that outputs a signal corresponding to the biological information of the subject, the sensor being disposed, when mounted, at at least one of between the finger of the subject and the bag and between the finger of the subject and the second support.

11. The support tool according to claim 10, comprising a tube connected to the channel formation part and having higher softness than the first support and the second support, wherein the tube comprises:

a ventilation channel communicating with the channel formation part to form a part of the fluid channel; and

a signal line housing part that houses a signal line electrically connected to the sensor.

12. The support tool according to claim 10, comprising a tube connected to the channel formation part, the tube having higher softness than the first support and the second support, wherein the tube comprises:

a ventilation channel communicating with the channel formation part to form a part of the fluid channel; and

a signal line retention part that retains, in a removable manner, a signal line electrically connected to the sensor.

13. The support tool according to any one of claims 10 to 12, wherein the biological information includes at least one of a capillary refill time and a blood substance concentration.

14. A biological information acquisition system comprising:

a support tool to be mounted on a finger of a subject;

a sensor to be mounted on the finger of the subject to output a signal corresponding to biological information of the subject;

an information acquisition part which acquires the biological information based on the signal output from the sensor; and

an information output part which outputs the information acquired by the information acquisition part,

wherein the support tool comprises:

a first support to be disposed, when mounted, on a first side of the finger of the subject, the first support comprising an inner peripheral face;

a second support to be disposed, when mounted, on a second side of the finger of the subject opposite to the first side, a relative position of the second support with respect to the first support being fixed;

a bag made of a material softer than the first support and the second support, the bag being at least partially disposed along the inner peripheral face; and

a channel formation part forming a fluid channel communicating with an inside of the bag,

wherein the sensor is disposed, when the support tool is mounted, at at least one of between the finger of the subject and the bag and between the finger of the subject and the second support, and

wherein the biological information acquisition system further comprises a pressure adjustment part which adjusts, through the channel formation part, a volume of a fluid inside the bag to adjust a pressure applied to the finger of the subject.

15. The biological information acquisition system according to claim 14, wherein the pressure adjustment part comprises a motor pump.

16. The biological information acquisition system according to claim 15, wherein the support tool comprises a switch which is actuated when mounted on the finger of the subject, and

wherein the biological information acquisition system further comprises a control part which drives the motor pump in accordance with the actuation of the switch.

17. The biological information acquisition system according to claim 15 or 16, comprising a power supply monitoring part which monitors a power feeding performance from a power supply to the motor pump,

wherein the power supply monitoring part controls the pressure adjustment part to stop driving the motor pump when the power feeding performance is lower than a predetermined value.

18. The biological information acquisition system according to any one of claims 14 to 17, wherein the pressure adjustment part includes a mechanism to manually adjust the volume of the fluid.

19. The biological information acquisition system according to any one of claims 14 to 18, comprising:

a pressure sensor which detects a pressure applied to the finger of the subject; and
an audio output part which outputs a sound corresponding to the pressure.

20. The biological information acquisition system according to any one of claims 14 to 19, wherein the sensor comprises:

a light emitter which emits first light having a first wavelength, and second light having a second wavelength; and
a light receiver which outputs a first signal and a second signal respectively in accordance with an intensity of the first light and an intensity of the second light which have been transmitted through or reflected on the finger of the subject, and

wherein the information acquisition part comprises:

a first light attenuation acquisition portion which acquires a light attenuation of the first light based on the first signal, and acquires a light attenuation of the second light based on the second signal;
a second light attenuation acquisition portion which acquires a blood-based light attenuation based on the light attenuation of the first light and the light attenuation of the second light; and
a capillary refill time specifying portion which specifies a capillary refill time in a tissue of the finger based on a temporal change of the blood-based light attenuation due to a compression of the finger of the subject by the bag.

21. The biological information acquisition system according to claim 20, wherein the light emitter emits third light having a third wavelength,
wherein the light receiver outputs a third signal in accordance with an intensity of the third light which has been transmitted through or reflected on the finger of the subject,
wherein the first wavelength and the second wavelength are wavelengths which are absorbed by hemoglobin,
wherein the third wavelength is a wavelength at which an absorption by water is larger than an absorption by hemoglobin; and
wherein the information acquisition part acquires information on a non-blood tissue of the subject based on at least a temporal change of the third signal generated due to the compression of the finger of the subject by the bag.

22. The biological information acquisition system according to any one of claims 14 to 19, wherein the sensor comprises:

a light emitter which emits lights having N kinds of wavelengths that are different from one another, N being an integer equal to or greater than 3; and
a light receiver which outputs N kinds of signals in accordance with intensities of the N kinds of light which have been transmitted through or reflected on the finger of the subject; and

wherein the information acquisition part comprises:

a first light attenuation acquisition portion which acquires N kinds of light attenuations based on the N kinds of signals;
a second light attenuation acquisition portion which acquires at most N-1 kinds of blood-based light attenuations based on at most N-1 kinds of combinations of two light attenuations selected from the N kinds of light attenuations; and
a blood substance concentration specifying portion which specifies at most N-1 kinds of blood substance concentrations based on the at most N-1 kinds of blood-based light attenuations.

23. The biological information acquisition system according to claim 22, wherein the information output part outputs the blood substance concentrations in a state in which the finger of the subject is not compressed by the bag,
wherein, while the finger of the subject is being compressed by the bag, the information output part either suspends outputting the blood substance concentrations or keeps pre-pressurization output values of the blood substance concentrations.

24. The biological information acquisition system according to any one of claims 14 to 23, comprising a control part which operates the pressure adjustment part at a predetermined interval.

25. The biological information acquisition system according to any one of claims 14 to 24, comprising a switch which starts an operation of the pressure adjustment part; and
a prohibition control part which invalidates an operation of the switch until a predetermined time elapses after the compression of the finger of the subject by the bag is released.

26. The biological information acquisition system according to claim 25, wherein the predetermined time is variable based on an operating state of the pressure adjustment part.

27. The biological information acquisition system according to any one of claims 14 to 26, comprising a pressure sensor which detects an internal pressure of the bag,
wherein the pressure adjustment part adjusts the volume of the fluid inside the bag based on a detection result of the pressure sensor so that the internal pressure becomes a target value.

FIG. 1

EP 3 117 769 A1

*FIG. 2*

*FIG. 3*

*FIG. 4*

## FIG. 5

## FIG. 6

*FIG. 7*

*FIG. 8*

## FIG. 9

# FIG. 10

UP

REAR

LEFT

RIGHT

FRONT

DOWN

*FIG. 11*

UP

REAR

LEFT — RIGHT

FRONT

DOWN

*FIG. 12*

UP

REAR

LEFT — RIGHT

FRONT

DOWN

# FIG. 13

FIG. 14

EP 3 117 769 A1

## FIG. 15

UP

REAR ← → RIGHT

LEFT ← → FRONT

DOWN

# FIG. 16

UP

RIGHT

REAR ← → FRONT

LEFT

DOWN

## INTERNATIONAL SEARCH REPORT

| | International application No. |
|---|---|
| | PCT/JP2015/055765 |

**A. CLASSIFICATION OF SUBJECT MATTER**
*A61B5/1455*(2006.01)i

According to International Patent Classification (IPC) or to both national classification and IPC

**B. FIELDS SEARCHED**

Minimum documentation searched (classification system followed by classification symbols)
A61B5/1455

Documentation searched other than minimum documentation to the extent that such documents are included in the fields searched
Jitsuyo Shinan Koho          1922–1996    Jitsuyo Shinan Toroku Koho    1996–2015
Kokai Jitsuyo Shinan Koho    1971–2015    Toroku Jitsuyo Shinan Koho    1994–2015

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | Microfilm of the specification and drawings annexed to the request of Japanese Utility Model Application No. 181640/1985 (Laid-open No. 90607/1987) (Omron Tateisi Electronics Co.), 10 June 1987 (10.06.1987), specification, page 6, line 6 to page 8, line 9; fig. 1 to 7 & US 4850369 A | 1-2,10 |
| X | JP 61-238225 A (Omron Tateisi Electronics Co.), 23 October 1986 (23.10.1986), page 2, upper left column, line 15 to page 3, upper left column, line 6; fig. 1 to 4 & US 4771790 A | 1-2,6-7,10, 14-15,18-19, 24,27 |
| Y | | 8-9,11-13, 16-17,20-23, 25 |
| A | | 3-5,26 |

☒ Further documents are listed in the continuation of Box C.    ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 08 May 2015 (08.05.15) | 19 May 2015 (19.05.15) |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (July 2009)

**INTERNATIONAL SEARCH REPORT**

| International application No. |
|---|
| PCT/JP2015/055765 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2007-144130 A  (Konica Minolta Sensing, Inc.), 14 June 2007 (14.06.2007), paragraphs [0036] to [0040], [0056] & US 2007/0100221 A1 | 9,13,16 |
| Y | JP 2012-115640 A  (Nihon Kohden Corp.), 21 June 2012 (21.06.2012), paragraphs [0001] to [0002], [0026] to [0064]; fig. 1 to 9 & US 2012/0130211 A1    & EP 2449965 A1 & CN 102525444 A | 9,13,20-23, 25 |
| Y | JP 2004-230000 A  (Nihon Kohden Corp.), 19 August 2004 (19.08.2004), paragraphs [0007] to [0020] & US 2004/0176670 A1 | 20-23 |
| Y | JP 2008-194488 A  (Nihon Kohden Corp.), 28 August 2008 (28.08.2008), paragraphs [0035] to [0047] & US 2002/0111748 A1 | 20-23 |
| Y | WO 2010/082444 A1  (Konica Minolta Sensing, Inc.), 22 July 2010 (22.07.2010), paragraphs [0031] to [0032]; fig. 2 (Family: none) | 8 |
| Y | JP 2006-102258 A  (Nippon Telegraph and Telephone Corp.), 20 April 2006 (20.04.2006), paragraphs [0016] to [0017]; fig. 2 (Family: none) | 11-12 |
| Y | JP 63-274845 A  (Fuji Photo Film Co., Ltd.), 11 November 1988 (11.11.1988), page 1, lower right column, lines 5 to 8 (Family: none) | 17 |
| Y | JP 2011-67238 A  (Mitsuba Corp.), 07 April 2011 (07.04.2011), paragraph [0062] (Family: none) | 25 |
| P,A | JP 2015-127 A  (Nihon Kohden Corp.), 15 January 2015 (15.01.2015), paragraphs [0022] to [0041]; fig. 1 to 3 & US 2014/0371557 A1    & CN 104224192 A | 20-23 |

Form PCT/ISA/210 (continuation of second sheet) (July 2009)

## INTERNATIONAL SEARCH REPORT

| International application No. |
| --- |
| PCT/JP2015/055765 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2000-166884 A  (NEC Medical Systems Kabushiki Kaisha), 20 June 2000 (20.06.2000), paragraphs [0010] to [0032]; fig. 1 to 5 & US 7524291 B1 | 3-5 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8082017 B2

- JP 2014047755 A